# NEW EUROPEAN PATENT SPECIFICATION

(11) **EP 2 688 587 B2**
(45) Date of publication and mention of the opposition decision: **16.08.2023**
(45) Mention of the grant of the patent: 29.07.2020
(21) Application number: 12710084.0
(22) Date of filing: 22.03.2012
(51) Int. Cl.: A61K 39/12, C12N 7/00, C12N 7/04

(54) **SALMONID ALPHAVIRUS VACCINE**
IMPFSTOFF GEGEN DAS LACHS-ALPHAVIRUS
VACCIN CONTRE LE ALPHAVIRUS DU SAUMON

(30) Priority: 25.03.2011 EP 11159807
(43) Date of publication of application: 29.01.2014
(73) Proprietor: Intervet International B.V., 5831 AN Boxmeer (NL)
(72) Inventor: PIJLMAN, Gorben, NL-6708 PB Wageningen (NL); METZ, Stefan, NL-6708 PB Wageningen (NL)
(74) Representative: Intervet International B.V.
(86) International application number: PCT/EP2012/055104
(87) International publication number: WO 2012/130723

(56) References cited:
- WO-A2-99/58639
- WO-A2-2010/062396
- MORIETTE CORALIE ET AL: "Recovery of a recombinant Salmonid alphavirus fully attenuated and protective for rainbow trout", JOURNAL OF VIROLOGY, vol. 80, no. 8, April 2006 (2006-04), pages 4088-4098, XP002657243, ISSN: 0022-538X
- LYNN DWIGHT E: "Comparison of cell line maintenance procedures on insect cells used for producing baculoviruses", IN VITRO CELLULAR AND DEVELOPMENTAL BIOLOGY ANIMAL, vol. 35, no. 5, May 1999 (1999-05), pages 248-251, XP002657244, ISSN: 1071-2690
- Stefan W. Metz ET AL: "Low Temperature-Dependent Salmonid Alphavirus Glycoprotein Processing and Recombinant Virus-Like Particle Formation", PLoS ONE, vol. 6, no. 10, 3 October 2011 (2011-10-03), page e25816, XP055211353, DOI: 10.1371/journal.pone.0025816
- ARUN K. DHAR: "Challenges and Opportunities in Developing Oral Vaccines against Viral Diseases of Fish", JOURNAL OF MARINE SCIENCE: RESEARCH & DEVELOPMENT, vol. 3, no. 1, 1 January 2013 (2013-01-01) , XP55501153, DOI: 10.4172/2155-9910.S1-003
- C.W.E. EMBREGTS ET AL: "Vaccination of carp against SVCV with an oral DNA vaccine or an insect cells-based subunit vaccine", FISH AND SHELLFISH IMMUNOLOGY, 1 March 2018 (2018-03-01), XP55501166, GB ISSN: 1050-4648, DOI: 10.1016/j.fsi.2018.03.028
- WATARU AKAHATA ET AL: "A virus-like particle vaccine for epidemic Chikungunya virus protects nonhuman primates against infection", NATURE MEDICINE, NATURE PUBLISHING GROUP, GB , vol. 16, no. 3 1 March 2010 (2010-03-01), pages 334-338, XP002676205, ISSN: 1546-170X, DOI: 10.1038/NM.2105 Retrieved from the Internet: URL:http://www.nature.com/nm/journal/v16/n 3/full/nm.2105.html [retrieved on 2010-01-28]

### SEQUENCE LISTING

<110> Intervet International b.v.
<120> Pancreas Disease virus vaccine
<130> 2011.003
<160> 2
<170> PatentIn version 3.5
<210> 1
   <211> 8716
   <212> DNA
   <213> salmonid alphavirus
<400> 1
   gacgcgccct gtagcggcgc attaagcgcg gcgggtgtgg tggttacgcg cagcgtgacc 60
   gctacacttg ccagcgccct agcgcccgct cctttcgctt tcttcccttc ctttctcgcc 120
   acgttcgccg gctttccccg tcaagctcta aatcgggggc tccctttagg gttccgattt 180
   agtgctttac ggcacctcga ccccaaaaaa cttgattagg gtgatggttc acgtagtggg 240
   ccatcgccct gatagacggt ttttcgccct ttgacgttgg agtccacgtt ctttaatagt 300
   ggactcttgt tccaaactgg aacaacactc aaccctatct cggtctattc ttttgattta 360
   taagggattt tgccgatttc ggcctattgg ttaaaaaatg agctgattta acaaaaattt 420
   aacgcgaatt ttaacaaaat attaacgttt acaatttcag gtggcacttt tcggggaaat 480
   gtgcgcggaa cccctatttg tttatttttc taaatacatt caaatatgta tccgctcatg 540
   agacaataac cctgataaat gcttcaataa tattgaaaaa ggaagagtat gagtattcaa 600
   catttccgtg tcgcccttat tccctttttt gcggcatttt gccttcctgt ttttgctcac 660
   ccagaaacgc tggtgaaagt aaaagatgct gaagatcagt tgggtgcacg agtgggttac 720
   atcgaactgg atctcaacag cggtaagatc cttgagagtt ttcgccccga agaacgtttt 780
   ccaatgatga gcacttttaa agttctgcta tgtggcgcgg tattatcccg tattgacgcc 840
   gggcaagagc aactcggtcg ccgcatacac tattctcaga atgacttggt tgagtactca 900
   ccagtcacag aaaagcatct tacggatggc atgacagtaa gagaattatg cagtgctgcc 960
   ataaccatga gtgataacac tgcggccaac ttacttctga caacgatcgg aggaccgaag 1020
   gagctaaccg cttttttgca caacatgggg gatcatgtaa ctcgccttga tcgttgggaa 1080
   ccggagctga atgaagccat accaaacgac gagcgtgaca ccacgatgcc tgtagcaatg 1140
   gcaacaacgt tgcgcaaact attaactggc gaactactta ctctagcttc ccggcaacaa 1200
   ttaatagact ggatggaggc ggataaagtt gcaggaccac ttctgcgctc ggcccttccg 1260
   gctggctggt ttattgctga taaatctgga gccggtgagc gtgggtctcg cggtatcatt 1320
   gcagcactgg ggccagatgg taagccctcc cgtatcgtag ttatctacac gacggggagt 1380
   caggcaacta tggatgaacg aaatagacag atcgctgaga taggtgcctc actgattaag 1440
   cattggtaac tgtcagacca agtttactca tatatacttt agattgattt aaaacttcat 1500
   ttttaattta aaaggatcta ggtgaagatc ctttttgata atctcatgac caaaatccct 1560
   taacgtgagt tttcgttcca ctgagcgtca gaccccgtag aaaagatcaa aggatcttct 1620
   tgagatcctt tttttctgcg cgtaatctgc tgcttgcaaa caaaaaaacc accgctacca 1680
   gcggtggttt gtttgccgga tcaagagcta ccaactcttt ttccgaaggt aactggcttc 1740
   agcagagcgc agataccaaa tactgtcctt ctagtgtagc cgtagttagg ccaccacttc 1800
   aagaactctg tagcaccgcc tacatacctc gctctgctaa tcctgttacc agtggctgct 1860
   gccagtggcg ataagtcgtg tcttaccggg ttggactcaa gacgatagtt accggataag 1920
   gcgcagcggt cgggctgaac ggggggttcg tgcacacagc ccagcttgga gcgaacgacc 1980
   tacaccgaac tgagatacct acagcgtgag cattgagaaa gcgccacgct tcccgaaggg 2040
   agaaaggcgg acaggtatcc ggtaagcggc agggtcggaa caggagagcg cacgagggag 2100
   cttccagggg gaaacgcctg gtatctttat agtcctgtcg ggtttcgcca cctctgactt 2160
   gagcgtcgat ttttgtgatg ctcgtcaggg gggcggagcc tatggaaaaa cgccagcaac 2220
   gcggcctttt tacggttcct ggccttttgc tggccttttg ctcacatgtt ctttcctgcg 2280
   ttatcccctg attctgtgga taaccgtatt accgcctttg agtgagctga taccgctcgc 2340
   cgcagccgaa cgaccgagcg cagcgagtca gtgagcgagg aagcggaaga gcgcctgatg 2400
   cggtattttc tccttacgca tctgtgcggt atttcacacc gcagaccagc cgcgtaacct 2460
   ggcaaaatcg gttacggttg agtaataaat ggatgccctg cgtaagcggg tgtgggcgga 2520
   caataaagtc ttaaactgaa caaaatagat ctaaactatg acaataaagt cttaaactag 2580
   acagaatagt tgtaaactga aatcagtcca gttatgctgt gaaaaagcat actggacttt 2640
   tgttatggct aaagcaaact cttcattttc tgaagtgcaa attgcccgtc gtattaaaga 2700
   ggggcgtggc caagggcatg gtaaagacta tattcgcggc gttgtgacaa tttaccgaac 2760
   aactccgcgg ccgggaagcc gatctcggct tgaacgaatt gttaggtggc ggtacttggg 2820
   tcgatatcaa agtgcatcac ttcttcccgt atgcccaact ttgtatagag agccactgcg 2880
   ggatcgtcac cgtaatctgc ttgcacgtag atcacataag caccaagcgc gttggcctca 2940
   tgcttgagga gattgatgag cgcggtggca atgccctgcc tccggtgctc gccggagact 3000
   gcgagatcat agatatagat ctcactacgc ggctgctcaa acctgggcag aacgtaagcc 3060
   gcgagagcgc caacaaccgc ttcttggtcg aaggcagcaa gcgcgatgaa tgtcttacta 3120
   cggagcaagt tcccgaggta atcggagtcc ggctgatgtt gggagtaggt ggctacgtct 3180
   ccgaactcac gaccgaaaag atcaagagca gcccgcatgg atttgacttg gtcagggccg 3240
   agcctacatg tgcgaatgat gcccatactt gagccaccta actttgtttt agggcgactg 3300
   ccctgctgcg taacatcgtt gctgctgcgt aacatcgttg ctgctccata acatcaaaca 3360
   tcgacccacg gcgtaacgcg cttgctgctt ggatgcccga ggcatagact gtacaaaaaa 3420
   acagtcataa caagccatga aaaccgccac tgcgccgtta ccaccgctgc gttcggtcaa 3480
   ggttctggac cagttgcgtg agcgcatacg ctacttgcat tacagtttac gaaccgaaca 3540
   ggcttatgtc aactgggttc gtgccttcat ccgtttccac ggtgtgcgtc acccggcaac 3600
   cttgggcagc agcgaagtcg aggcatttct gtcctggctg gcgaacgagc gcaaggtttc 3660
   ggtctccacg catcgtcagg cattggcggc cttgctgttc ttctacggca aggtgctgtg 3720
   cacggatctg ccctggcttc aggagatcgg aagacctcgg ccgtcgcggc gcttgccggt 3780
   ggtgctgacc ccggatgaag tggttcgcat cctcggtttt ctggaaggcg agcatcgttt 3840
   gttcgcccag gactctagct atagttctag tggttggcta cgtatactcc ggaatattaa 3900
   tagatcatgg agataattaa aatgataacc atctcgcaaa taaataagta ttttactgtt 3960
   ttcgtaacag ttttgtaata aaaaaaccta taaatattcc ggattattca taccgtccca 4020
   ccatcgggcg cggatcccgg tccgaagcgc gcggaattcg ccgccaccat gtttcccatg 4080
   caattcacaa actcagccta tcgccagatg gagcccatgt tcgcaccggg tcctcgagga 4140
   caagtacagc cgtaccggcc gcgcactaag cgtcgccaag agccgcaagt cggcaacgct 4200
   gctattgctg ccctcgcgaa ccagatgagc gcgctccagc tacaggtggc tggacttgcc 4260
   ggccaggcaa gggtggatcg tcgtggacca agacgtgttc agaaaagcaa gcagaagaag 4320
   aagaatccct ccaacggaga aaaacccaag gagaagaaga agaagcaaaa acaacaggag 4380
   aagaaaggga gcggtggcgt aatcaaaaaa ccacggaacc ggcccggaaa ggaggtaagg 4440
   atctccgtaa agcgtgcccg acagagcacc ttccccgtgt accatgacgg tgctatatcc 4500
   ggctacgctg tgctgattgg ttctcgcgtg tttaagccag cgcacgtgaa gggtaagatc 4560
   gaccaccccg agctggcgga catcaagttc caggtcgccg aggacatgga cctcgaagca 4620
   gctgcttacc ctaagagtat gcgagaccag gcggctgaac cagcaaccat gatggacgga 4680
   gtgtataact gggagtacgg cactatcaga gtggaggaca atgtcgtaat tgacgcgagc 4740
   ggcagaggta agccgggtga cagtggaagg gccattaccg acaactcagg aaaggttgtt 4800
   ggcattgtcc tcggaggggg acccgacggc aggcgcacac gcctctccgt gataggtttc 4860
   gacaagaaga tgaaggctag agagatcgcc tacagcgagg ccataccttg gacacgtgct 4920
   ccagccctcc tgctgctgcc catggtcatc gcctgtacct acaactccaa cacctttgat 4980
   tgctccaaac cgtcctgcca ggactgttgt atcactgctg aaccagagaa ggccatggct 5040
   atgttgaagg acaacctgaa cgacccgaac tattgggacc tactcattgc cgtcaccacc 5100
   tgcaattccg cccggaaaaa gagggctgtg tctgcgtcgc ctgccgccgt ttacgacaca 5160
   caaatcctcg ccgcccacgc agctgcctcc ccgtacaggg cgtattgccc cgactgtgat 5220
   ggaactgcct gcatctcgcc gatagctatt gacgaggtgg taagcagcgg tagcgaccac 5280
   gtcctccgca tccgggtcgg ttctcaatcg ggagtgaccg ctaaaggcgg tgcggcgggt 5340
   gaaacatctc tgcgatacct gggaagggac ggtaaggtgc acgccgcgga caacacgcgg 5400
   cttgtggtgc gcaccactgc aaagtgcgat gtgctgcagg ccaccggcca ctacatcctg 5460
   gccagctgcc cagaggggca gagtattact gttgcggcca cactggacgg cacccgccac 5520
   caatgcacca cggttttcga acatcaagta acggagaagt tcaccagaga acgcagcaag 5580
   ggccaccact tgtccgacct aaccaagaag tgcaccagat tttccaccac cccgaagaag 5640
   tccgccccct acctcgttga cgtgtacgac gctctgccga tttccgtaga gattagcacc 5700
   gttgtaacat gcaacgacaa tcagtgcaca gtgagggtgt cacccggtac cacagtgaaa 5760
   ttcgataaga agtgcaagag cgctgcccaa gcgaccgtta cctttaccag cgactcccag 5820
   acgtttacgt gtgaggagcc ggttctgacg gccgccagta tcacccaggg caagccgcac 5880
   cttagatcat ctatgttgcc cagcggaggc aaggaagtga aggcgaggat cccattcccg 5940
   ttcccgccag agaccgcgac ctgcagagta agtgtcgccc cgctgccgtc gatcacctat 6000
   gaggaaagcg acgttctgct ggccggtacc gcgaagtacc ccgtgctgct gactacacgg 6060
   aaccttggtt tccacagcaa tgccacatcc gaatggatcc agggtaagta cttgcgccgt 6120
   atcccggtca cgccccaagg gatcgaacta acgtggggaa ataacgcacc gttgcacttc 6180
   tggtcatctg ttaggtacgc atctggggac gccgacgcgt acccttggga acttctggtg 6240
   caccacacca agcaccatcc ggagtacgcg tgggcgtttg taggagttgc atgtggtctg 6300
   ctggttattg cagtatgcat gttcgcgtgc gcatgcaaca gagtgcggta ctctttggtc 6360
   gccaacacgt tcaacccgaa cccaccacca ctgaccgcac tgactgcagc attgtgctgc 6420
   atacctgggg ctcgtgcgga ccaaccctac ctggacatca ttgcctacct gtggaccaac 6480
   agcaaagtgg ccttcgggct gcaatgcgcg gcgcccgtgg cttgcgtgct catcgtcaca 6540
   tacgccctta gacactgcag actgtgctgc aagtcttttt taggggtaag agggtggtca 6600
   gctctgctgg tcatccttgc gtatgtacag agctgcaaga gctacgaaca caccgtggtg 6660
   gtcccaatgg acccgagagc cccgtcgtac gaagcagtga taaaccggaa tgggtatgat 6720
   cccttgaagc tgaccattgc agtgaacttt accgtcatct caccaactac agctctggaa 6780
   tactggactt gcgcaagtgt ccctatcgtc gagccgcccc atgtgggctg ctgcacgtcg 6840
   gtgtcctgcc cctccgacct ctccacactg cacgcgttca ccggcaaagc cgtctccgac 6900
   gtgcactgcg atgtgcacac aaacgtgtac cccttgttgt ggggtgcggc tcattgcttc 6960
   tgctccaccg agaatacaca agtcagcgcg gtggctgcca ccgtctctga attttgtgct 7020
   caggactcag agcgcgccga agcgtttagc gttcacagca gctcagtcac tgcagaggtt 7080
   ctggtgacgc ttggtgaagt ggtgacggca gtccacgttt acgtggacgg ggtaacatct 7140
   gccaggggta ccgacctcaa gatcgtggct gggccaataa caactgacta ctccccgttc 7200
   gatcgcaaag tagtccgtat cggcgaagag gtctataact acgactggcc tccttacggg 7260
   gctggtcgac caggcacatt cggggatatt caagctaggt caaccaacta tgtcaagccc 7320
   aatgatctgt acggggatat cggaatcgaa gtactgcagc cgactaatga ccatgtgcac 7380
   gtggcttaca cgtatacgac ctccgggtta ctgcgttggc tgcaggacgc tccgaagcca 7440
   cttagtgtca cagcaccgca cggttgtaag atcagtgcca acccgctcct ggccctcgat 7500
   tgtggggttg gcgccgtccc catgtccatc aacattccgg acgcgaagtt cacccgcaaa 7560
   ttgaaggatc cgaaaccatc ggccctgaaa tgcgtggtgg acagttgcga gtacggggtg 7620
   gactacgggg gcgccgccac gatcacctat gagggccacg aggctgggaa gtgcgggatc 7680
   cattccctga caccaggagt ccctctgaga acatcagtgg tcgaagtggt tgccggcgct 7740
   aacaccgtca aaacgacctt ctcctcaccc acgcccgagg tcacactcga ggtagagatc 7800
   tgttcggcaa tggtgacgtg cgccagtgag tgcactccac cgaaggaaca tgtagtcgca 7860
   accaggcctc gccatggcag cgacactgga ggctacatct ccgggcccgc aatgcgctgg 7920
   gccggtggga ttgtagggac cttagcggtc ctgttcctca tccttgccgt tacctactgc 7980
   gtggtgaaga agtgccgctc taaaagaatc cggatagtca agagctaagc ggccgctttc 8040
   gaatctagag cctgcagtct cgaggcatgc ggtaccaagc ttgtcgagaa gtactagagg 8100
   atcataatca gccataccac atttgtagag gttttacttg ctttaaaaaa cctcccacac 8160
   ctccccctga acctgaaaca taaaatgaat gcaattgttg ttgttaactt gtttattgca 8220
   gcttataatg gttacaaata aagcaatagc atcacaaatt tcacaaataa agcatttttt 8280
   tcactgcatt ctagttgtgg tttgtccaaa ctcatcaatg tatcttatca tgtctggatc 8340
   tgatcactgc ttgagcctag gagatccgaa ccagataagt gaaatctagt tccaaactat 8400
   tttgtcattt ttaattttcg tattagctta cgacgctaca cccagttccc atctattttg 8460
   tcactcttcc ctaaataatc cttaaaaact ccatttccac ccctcccagt tcccaactat 8520
   tttgtccgcc cacagcgggg catttttctt cctgttatgt ttttaatcaa acatcctgcc 8580
   aactccatgt gacaaaccgt catcttcggc tactttttct ctgtcacaga atgaaaattt 8640
   ttctgtcatc tcttcgttat taatgtttgt aattgactga atatcaacgc ttatttgcag 8700
   cctgaatggc gaatgg 8716
<210> 2
   <211> 1319
   <212> PRT
   <213> salmonid alphavirus
<400> 2
   Met Phe Pro Met Gln Phe Thr Asn Ser Ala Tyr Arg Gln Met Glu Pro
   1               5                   10                  15
   Met Phe Ala Pro Gly Pro Arg Gly Gln Val Gln Pro Tyr Arg Pro Arg
               20                  25                  30
   Thr Lys Arg Arg Gln Glu Pro Gln Val Gly Asn Ala Ala Ile Ala Ala
           35                  40                  45
   Leu Ala Asn Gln Met Ser Ala Leu Gln Leu Gln Val Ala Gly Leu Ala
       50                  55                  60
   Gly Gln Ala Arg Val Asp Arg Arg Gly Pro Arg Arg Val Gln Lys Ser
   65                  70                  75                  80
   Lys Gln Lys Lys Lys Asn Pro Ser Asn Gly Glu Lys Pro Lys Glu Lys
                   85                  90                  95
   Lys Lys Lys Gln Lys Gln Gln Glu Lys Lys Gly Ser Gly Gly Val Ile
               100                 105                 110
   Lys Lys Pro Arg Asn Arg Pro Gly Lys Glu Val Arg Ile Ser Val Lys
           115                 120                 125
   Arg Ala Arg Gln Ser Thr Phe Pro Val Tyr His Asp Gly Ala Ile Ser
       130                 135                 140
   Gly Tyr Ala Val Leu Ile Gly Ser Arg Val Phe Lys Pro Ala His Val
   145                 150                 155                 160
   Lys Gly Lys Ile Asp His Pro Glu Leu Ala Asp Ile Lys Phe Gln Val
                   165                 170                 175
   Ala Glu Asp Met Asp Leu Glu Ala Ala Ala Tyr Pro Lys Ser Met Arg
               180                 185                 190
   Asp Gln Ala Ala Glu Pro Ala Thr Met Met Asp Gly Val Tyr Asn Trp
           195                 200                 205
   Glu Tyr Gly Thr Ile Arg Val Glu Asp Asn Val Val Ile Asp Ala Ser
       210                 215                 220
   Gly Arg Gly Lys Pro Gly Asp Ser Gly Arg Ala Ile Thr Asp Asn Ser
   225                 230                 235                 240
   Gly Lys Val Val Gly Ile Val Leu Gly Gly Gly Pro Asp Gly Arg Arg
   245 250 255
   Thr Arg Leu Ser Val Ile Gly Phe Asp Lys Lys Met Lys Ala Arg Glu
   260 265 270
   Ile Ala Tyr Ser Glu Ala Ile Pro Trp Thr Arg Ala Pro Ala Leu Leu
   275 280 285
   Leu Leu Pro Met Val Ile Ala Cys Thr Tyr Asn Ser Asn Thr Phe Asp
   290 295 300
   Cys Ser Lys Pro Ser Cys Gln Asp Cys Cys Ile Thr Ala Glu Pro Glu
   305 310 315 320
   Lys Ala Met Ala Met Leu Lys Asp Asn Leu Asn Asp Pro Asn Tyr Trp
   325 330 335
   Asp Leu Leu Ile Ala Val Thr Thr Cys Asn Ser Ala Arg Lys Lys Arg
   340 345 350
   Ala Val Ser Ala Ser Pro Ala Ala Val Tyr Asp Thr Gln Ile Leu Ala
   355 360 365
   Ala His Ala Ala Ala Ser Pro Tyr Arg Ala Tyr Cys Pro Asp Cys Asp
   370 375 380
   Gly Thr Ala Cys Ile Ser Pro Ile Ala Ile Asp Glu Val Val Ser Ser
   385 390 395 400
   Gly Ser Asp His Val Leu Arg Ile Arg Val Gly Ser Gln Ser Gly Val
   405 410 415
   Thr Ala Lys Gly Gly Ala Ala Gly Glu Thr Ser Leu Arg Tyr Leu Gly
   420 425 430
   Arg Asp Gly Lys Val His Ala Ala Asp Asn Thr Arg Leu Val Val Arg
   435 440 445
   Thr Thr Ala Lys Cys Asp Val Leu Gln Ala Thr Gly His Tyr Ile Leu
   450 455 460
   Ala Ser Cys Pro Glu Gly Gln Ser Ile Thr Val Ala Ala Thr Leu Asp
   465 470 475 480
   Gly Thr Arg His Gln Cys Thr Thr Val Phe Glu His Gln Val Thr Glu
   485 490 495
   Lys Phe Thr Arg Glu Arg Ser Lys Gly His His Leu Ser Asp Leu Thr
               500                 505                 510
   Lys Lys Cys Thr Arg Phe Ser Thr Thr Pro Lys Lys Ser Ala Pro Tyr
           515                 520                 525
   Leu Val Asp Val Tyr Asp Ala Leu Pro Ile Ser Val Glu Ile Ser Thr
       530                 535                 540
   Val Val Thr Cys Asn Asp Asn Gln Cys Thr Val Arg Val Ser Pro Gly
   545                 550                 555                 560
   Thr Thr Val Lys Phe Asp Lys Lys Cys Lys Ser Ala Ala Gln Ala Thr
                   565                 570                 575
   Val Thr Phe Thr Ser Asp Ser Gln Thr Phe Thr Cys Glu Glu Pro Val
               580                 585                 590
   Leu Thr Ala Ala Ser Ile Thr Gln Gly Lys Pro His Leu Arg Ser Ser
           595                 600                 605
   Met Leu Pro Ser Gly Gly Lys Glu Val Lys Ala Arg Ile Pro Phe Pro
       610                 615                 620
   Phe Pro Pro Glu Thr Ala Thr Cys Arg Val Ser Val Ala Pro Leu Pro
   625                 630                 635                 640
   Ser Ile Thr Tyr Glu Glu Ser Asp Val Leu Leu Ala Gly Thr Ala Lys
                   645                 650                 655
   Tyr Pro Val Leu Leu Thr Thr Arg Asn Leu Gly Phe His Ser Asn Ala
               660                 665                 670
   Thr Ser Glu Trp Ile Gln Gly Lys Tyr Leu Arg Arg Ile Pro Val Thr
           675                 680                 685
   Pro Gln Gly Ile Glu Leu Thr Trp Gly Asn Asn Ala Pro Leu His Phe
       690                 695                 700
   Trp Ser Ser Val Arg Tyr Ala Ser Gly Asp Ala Asp Ala Tyr Pro Trp
   705                 710                 715                 720
   Glu Leu Leu Val His His Thr Lys His His Pro Glu Tyr Ala Trp Ala
                   725                 730                 735
   Phe Val Gly Val Ala Cys Gly Leu Leu Val Ile Ala Val Cys Met Phe
               740                 745                 750
   Ala Cys Ala Cys Asn Arg Val Arg Tyr Ser Leu Val Ala Asn Thr Phe
           755                 760                 765
   Asn Pro Asn Pro Pro Pro Leu Thr Ala Leu Thr Ala Ala Leu Cys Cys
       770                 775                 780
   Ile Pro Gly Ala Arg Ala Asp Gln Pro Tyr Leu Asp Ile Ile Ala Tyr
   785                 790                 795                 800
   Leu Trp Thr Asn Ser Lys Val Ala Phe Gly Leu Gln Cys Ala Ala Pro
                   805                 810                 815
   Val Ala Cys Val Leu Ile Val Thr Tyr Ala Leu Arg His Cys Arg Leu
               820                 825                 830
   Cys Cys Lys Ser Phe Leu Gly Val Arg Gly Trp Ser Ala Leu Leu Val
           835                 840                 845
   Ile Leu Ala Tyr Val Gln Ser Cys Lys Ser Tyr Glu His Thr Val Val
       850                 855                 860
   Val Pro Met Asp Pro Arg Ala Pro Ser Tyr Glu Ala Val Ile Asn Arg
   865                 870                 875                 880
   Asn Gly Tyr Asp Pro Leu Lys Leu Thr Ile Ala Val Asn Phe Thr Val
                   885                 890                 895
   Ile Ser Pro Thr Thr Ala Leu Glu Tyr Trp Thr Cys Ala Ser Val Pro
               900                 905                 910
   Ile Val Glu Pro Pro His Val Gly Cys Cys Thr Ser Val Ser Cys Pro
           915                 920                 925
   Ser Asp Leu Ser Thr Leu His Ala Phe Thr Gly Lys Ala Val Ser Asp
       930                 935                 940
   Val His Cys Asp Val His Thr Asn Val Tyr Pro Leu Leu Trp Gly Ala
   945                 950                 955                 960
   Ala His Cys Phe Cys Ser Thr Glu Asn Thr Gln Val Ser Ala Val Ala
                   965                 970                 975
   Ala Thr Val Ser Glu Phe Cys Ala Gln Asp Ser Glu Arg Ala Glu Ala
               980                 985                 990
   Phe Ser Val His Ser Ser Ser Val  Thr Ala Glu Val Leu  Val Thr Leu
   995 1000 1005
   Gly Glu Val Val Thr Ala Val His Val Tyr Val Asp Gly Val Thr
   1010 1015 1020
   Ser Ala Arg Gly Thr Asp Leu Lys Ile Val Ala Gly Pro Ile Thr
   1025 1030 1035
   Thr Asp Tyr Ser Pro Phe Asp Arg Lys Val Val Arg Ile Gly Glu
   1040 1045 1050
   Glu Val Tyr Asn Tyr Asp Trp Pro Pro Tyr Gly Ala Gly Arg Pro
   1055 1060 1065
   Gly Thr Phe Gly Asp Ile Gln Ala Arg Ser Thr Asn Tyr Val Lys
   1070 1075 1080
   Pro Asn Asp Leu Tyr Gly Asp Ile Gly Ile Glu Val Leu Gln Pro
   1085 1090 1095
   Thr Asn Asp His Val His Val Ala Tyr Thr Tyr Thr Thr Ser Gly
   1100 1105 1110
   Leu Leu Arg Trp Leu Gln Asp Ala Pro Lys Pro Leu Ser Val Thr
   1115 1120 1125
   Ala Pro His Gly Cys Lys Ile Ser Ala Asn Pro Leu Leu Ala Leu
   1130 1135 1140
   Asp Cys Gly Val Gly Ala Val Pro Met Ser Ile Asn Ile Pro Asp
   1145 1150 1155
   Ala Lys Phe Thr Arg Lys Leu Lys Asp Pro Lys Pro Ser Ala Leu
   1160 1165 1170
   Lys Cys Val Val Asp Ser Cys Glu Tyr Gly Val Asp Tyr Gly Gly
   1175 1180 1185
   Ala Ala Thr Ile Thr Tyr Glu Gly His Glu Ala Gly Lys Cys Gly
   1190 1195 1200
   Ile His Ser Leu Thr Pro Gly Val Pro Leu Arg Thr Ser Val Val
   1205 1210 1215
   Glu Val Val Ala Gly Ala Asn Thr Val Lys Thr Thr Phe Ser Ser
   1220 1225 1230
   Pro Thr  Pro Glu Val Thr Leu  Glu Val Glu Ile Cys  Ser Ala Met
       1235                 1240                 1245
   Val Thr  Cys Ala Ser Glu Cys  Thr Pro Pro Lys Glu  His Val Val
       1250                 1255                 1260
   Ala Thr  Arg Pro Arg His Gly  Ser Asp Thr Gly Gly  Tyr Ile Ser
       1265                 1270                 1275
   Gly Pro  Ala Met Arg Trp Ala  Gly Gly Ile Val Gly  Thr Leu Ala
       1280                 1285                 1290
   Val Leu  Phe Leu Ile Leu Ala  Val Thr Tyr Cys Val  Val Lys Lys
       1295                 1300                 1305
   Cys Arg  Ser Lys Arg Ile Arg  Ile Val Lys Ser
       1310                 1315

## Description

The present invention and the scope of protection is defined by the appended claims. It relates to Salmonid Alphavirus (SAV) antigens, to their use as a medicament, to baculovirus expression vectors comprising a polynucleotide encoding the SAV E2 protein and insect cells comprising such expression vectors, to the use of baculovirus based expression systems for the production of SAV antigens, to methods for the production of SAV antigens, and to vaccines comprising such SAV antigens.

The fish pathogenic virus Salmon Pancreas Disease virus (SPDV) causes a serious disease in salmon and trout. The symptoms of the disease caused by SPDV in salmon and trout differ slightly. The symptoms caused by SPDV in trout led to the name sleeping disease. Therefore, the SPDV isolated from trout is also known under the name Sleeping Disease virus (SDV). The various isolates are cross-protective, and antibodies to one of the subtypes show cross-reaction with the other subtypes.

SPDV has been described to belong to the genus alphavirus, in the family Togaviridae (Villoing et al., 2000, J. of Virol., vol. 74, p. 173-183). Several closely related subtypes of the virus have been described. The virus is nowadays more commonly known as Salmonid Alphavirus (SAV) and was found to be separate from the groups around the terrestrial Sindbis-like and encephalitis-type Alphaviruses (Powers et al., 2001, J. of Virol., vol. 75, p. 10118-10131). (Weston et al., 2002, J. of Virol., vol. 76, p. 6155-6163).

A subdivision of the SAV species has been proposed recently (Weston et al., 2005, Dis. of Aq. Organ., vol. 66, p. 105-111; Hodneland et al., 2005, Dis. of Aq. Organ., vol. 66, p. 113-120), wherein i.e. SAV subtype 1 is formed by the viruses having resemblance to the SPDV isolates from Ireland and UK; subtype 2 is formed by the SDV isolates; and subtype 3 is formed by the Norwegian isolates of SPDV.

The aquaculture industry producing salmon or trout, suffers considerably from outbreaks of SAV, which cause reduced growth, and mortality of between 10-60 percent of the animals. Research has led to the development of vaccines (i.a. as described in European Patent EP 712,926), and Norvax^{®} Compact PD, an inactivated SAV virus vaccine, is commercially available for several years now for immunisation of salmon against SPDV. Vaccines based on SAV viral envelope protein E2 have also been described (EP 1,075,523).

Inactivated whole SAV vaccines are known to be effective. However, the production of inactivated whole virus is not very efficient due to the relatively low virus titres obtained. Moreover, it requires careful inactivation of the virus without diminishing viral immunogenicity. Also meticulous quality control of the viral inactivation process is required, to assure that no live virus remains. The same is true for subunit vaccines based on proteins isolated from viral cultures. Consequently, production of such vaccines comes at a certain price.

An improvement is the production of subunit vaccines in a recombinant expression system, where no pathogenic virus is used. However, compared to subunit vaccines, inactivated whole virus vaccines present the viral structural proteins in a more natural context.

Consequently there is a need for alternative SAV vaccines that combine the immunogenicity of a whole virus vaccine with the safety of a subunit vaccine. Such alternative vaccines are known for mammalian members of the Alphavirus family such as e.g. the Chikungunya virus in the form of Virus Like Particles (VLP).

Alphaviruses come into being when the cytoplasmic nucleocapsid core, comprising the viral genomic RNA leaves the cell through the host cell membrane. The virus, after this budding process, carries i.a. the structural proteins E1 and E2, that are present at the host cell membrane as a result of the whole viral infection cycle.

Virus-like particles structurally resemble infective alphavirus particles, but they are non-infectious in the sense that they do not contain viral genetic material, or at least not all of the viral genetic material that is necessary for the virus to produce infective progeny virus. In many cases, a virus-like particle has one or more deficiencies in one or more of the non-structural genes. Also, in principle VLP's need not necessarily carry all structural proteins, such as envelope proteins and other proteins present on the surface of wild type virus. In principle, if a truncated E1-gene would be expressed that is not encoding a full-length E1 envelope protein (e.g. as a result of a deletion in the E1-gene), a full-length E1 envelope protein would consequently not be present on the cell membrane of a cell expressing that truncated E1 envelope protein. Nevertheless, in such cases a virus-like particle could still be formed when the core leaves the cell through the cell membrane, albeit that this virus-like particle would not be carrying a full-length E1 envelope protein at its surface. Chikungunya virus VLP's and methods for their production have been described i.a. in International Patent Application WO 2010/062396. In WO 2010/062396, a mammalian cell expression system and mammalian expression vectors were used for the production of Chikungunya VLP's. Other expression systems such as baculovirus based systems were suggested.

Amongst the eukaryotic cell expression systems, the baculovirus based expression systems are attractive systems, because they, on the average, have an expression level that exceeds that of e.g. mammalian cells.

It turns out, however, that it is not possible to produce immunogenic Salmonid Alphavirus VLP's in baculovirus based expression systems under the conditions known in the art for baculovirus based expression systems. It was unexpectedly found that at the temperature of 27 °C as generally used in the art, for unknown reasons, no immunogenic E2-protein appeared at the surface of *Spodoptera frugiperda* cells that were infected with a recombinant baculovirus comprising the polynucleotide encoding the Salmonid Alphavirus (SAV) Capsid protein, E3 protein, E2 envelope protein, 6K protein and E1 envelope protein under the control of a baculovirus promoter.

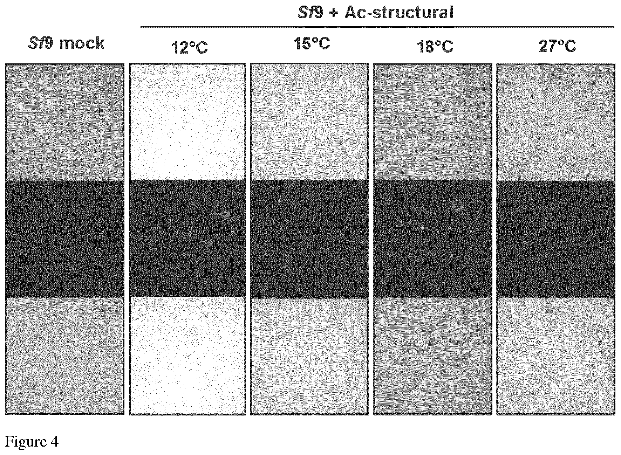

And since no immunogenic E2 is present on the cell surface, no immunogenic VLP's were formed. See Example 1, figure 4, in the last column, marked "27 °C", where Spodoptera frugiperda cells infected with the recombinant baculovirus were subjected to immunostaining with a SAV E2-specific monoclonal antibody. In the middle picture of that last column, it can be seen that no E2-specific staining is detectable.

It is an objective of the present invention to provide methods that do allow expression of immunogenic Salmonid Alphavirus VLP's in baculovirus based expression systems.

It was surprisingly found now, that such immunogenic SAV VLP's can however be made in baculosystem expression systems, if such systems are used at temperatures that are far below the optimal temperature for baculovirus expression generally used in the art.

In practice this means that such temperatures are significantly below the standard temperature of 27 °C. Such significantly lower temperatures would usually be in the range of 12-22 degrees Celsius (°C), preferably in the range of 12-20 °C. Methods for the production of VLP's in baculovirus based expression systems will be discussed (*vide infra*).

In Example 1, in figure 4, in the columns marked "12 °C", "15 °C" and "18 °C" it can be seen that indeed at these temperatures, on the surface of *Spodoptera frugiperda* cells infected with the recombinant baculovirus a clear ring-shaped E2-specific staining now is detectable.

The present invention and the scope of protection is defined by the appended claims.

Thus, a first embodiment of the present invention relates to an immunogenic Salmonid Alphavirus-like particle (immunogenic SAV VLP) directly obtained by a method for the production of an immunogenic SAV VLP in a baculovirus based expression system, said method comprising:
(a) Infecting insect cells with said baculovirus expression vector either
   (i) at a temperature of 12°C-22°C, or
   (ii) at a temperature of 22°C-27°C followed by lowering the temperature of the cell culture to 12°C-22°C
(b) growing cells at a temperature of 12°C-22°C
(c) optionally isolating the VLPs from the culture medium wherein the baculovirus expression vector comprises at least genetic information encoding an Alphavirus Capsid protein. E3 envelope protein, and an E2 envelope protein, 6K protein and E1 envelope protein, wherein at least the E2 and the E3 proteins are a Salmonid Alphavirus (SAV) E2 and E3 envelope proteins.

Salmonid Alphaviruses have a single-stranded positive RNA genome of approximately 12 kb that contains a 3' poly-A tail and is capped at the 5' end. It encodes two open reading frames (ORF). The non-structural ORF is directly translated from the viral RNA into the non-structural polyprotein which is processed into the mature non-structural proteins: nsP1-nsP4. The structural ORF is first transcribed into a 26S subgenomic RNA and is translated into the structural polyprotein, which is processed into the mature structural proteins: Capsid (C), E3, E2, 6K and E1 (McLoughlin and Graham, 2007) (Strauss and Strauss, 1994).
The viral RNA is encapsidated by a 40 nm icosahedral nucleocapsid composed of 240 copies of C and is enveloped by a host derived lipid bilayer. The two viral glycoproteins (E1 and E2) are embedded in the envelope and are exposed on the surface of the virion as trimeric spikes. E2 (50 kDa) contains the major neutralizing epitopes and is responsible for receptor recognition and binding, whereas E1 (55 kDa) regulates the fusion of the viral envelope with the endosomal membranes (Kuhn, 2007) The trimeric spikes contain three interacting E1-E2 heterodimers and are anchored in the lipid envelope with their C-terminal end. Including the trimeric spikes, the diameter of one virion is approximately 69 nm (Strauss and Strauss, 1994) (Kuhn, 2007).

The structural proteins are translated from a subgenomic mRNA and are expressed as one polyprotein in a C-E3-E2-6K-E1 arrangement. The structural polyprotein is both co- and post-translationally processed. The capsid protein is 30kDa in size and forms the nucleocapsid, which protects the genomic RNA against degradation. It has a C-terminal serine protease activity that acts *in cis* to release itself from the nascent polyprotein.
Once C has been cleaved, N-terminal signal sequences in E3 and 6K translocate the remaining polyprotein to the endoplasmic reticulum (ER). Two hydrophobic stretches are located at the C-terminus of both E2 and E1, which function as transmembrane anchors. Both E3 and 6K are important for the correct folding of the nascent glycoproteins. In the lumen of the ER, host signalase cleaves between E2-6K and between 6K-E1, yielding E3E2, 6K and E1. Folding of the glycoproteins starts in the ER and depends on molecular chaperones, folding enzymes, energy and the formation of disulfide bonds Furthermore, carbohydrate chains are added to E2 and E1. Just before reaching the cell surface, host furin-dependent cleavage releases E3 from E2, which results in mature E1/E2 trimeric heterodimer spikes, that are expressed at the plasma membrane of infected cells (Kuhn, 2007).

A SAV VLP in its minimal form would at least comprise the nucleocapsid formed by the capsid protein and the E2-protein.

Thus, in order to produce immunogenic SAV VLP's, it is necessary to allow for the expression of at least, next to the capsid protein, the genetic information encoding the Salmonid Alphavirus (SAV) E2 envelope protein. For correct folding/expression of the E2 envelope protein and the E3 protein is also expressed.

A SAV VLP according to the invention comprises all structural proteins, as is the case for wild-type PD-virus.

Thus, all structural proteins of the virus are expressed: the Alphavirus Capsid protein, E3 protein, E2 envelope protein, 6K protein and E1 envelope protein. Of these, at least the E2 and the E3 envelope proteins should be the Salmonid Alphavirus E2 envelope protein (*Vide infra*).

This can i.a. be done by expressing the genetic information for each of the following gene combinations: 6K/E1, E3E2 and C. This goal can i.a. be obtained by cloning genetic information encoding respectively 6K/E1, E3E2 and C in a baculovirus expression vector under the control of e.g. the polyhedrin promoter, the GP64 promoter, the P6.9 promoter or the P10 promoter. These baculoviral promoters are well-known in the art.

Preferably, this is done in an alternative way; by cloning a polynucleotide encoding Capsid protein, E3 protein, E2 envelope protein, 6K protein and E1 envelope protein under the control of a baculovirus promoter, followed by subsequent expression of the polyprotein comprising the Capsid protein, E3 protein, E2 envelope protein, 6K protein and E1 envelope protein.
This approach mimics the way in which wild-type alphavirus is replicated: during the replication cycle this polyprotein is formed and auto-processed into the various proteins.

Theoretically, the Capsid protein, 6K protein and E1 envelope protein may originate from another, non-SAV alphavirus such as e.g. Sindbis virus, Semliki forest virus, Equine Encephalitis virus or Chikungunya virus. The E2 envelope protein, and the immediately upstream located E3 protein are that of SAV, because it is the E2 envelope protein that is responsible for the induction of the main immunological response against SAV-infection.

More preferably, all structural proteins present at the SAV VLP are SAV proteins.
Therefore, more preferably, the structural genes are expressed from a SAV polynucleotide.

Preferably, the SAV E2 envelope protein or the SAV polynucleotide is selected from the group existing of SAV 1, SAV2, SAV3, SAV4, SAV5, and SAV6.

More preferably the SAV E2 envelope protein or the SAV polynucleotide is the E2 envelope protein of the polynucleotide of SAV1 or SAV3.

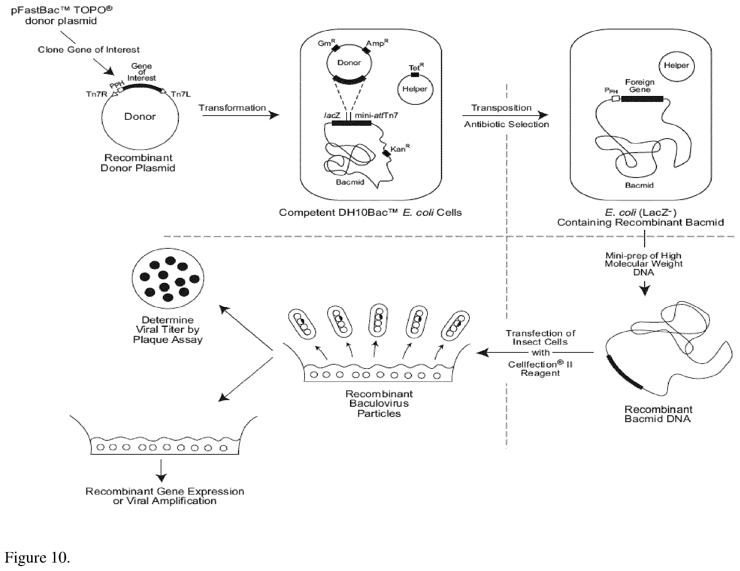

One of the most common used expression methods in baculovirus based expression systems is the Bac-to-Bac expression system from Invitrogen, which uses site-specific transposition of an expression cassette into the baculovirus genome, which can be maintained and manipulated in *E. coli* (Luckow et.al, 2003) (Figure 10).
If this system is used, the foreign genetic information, e.g. the E3E2 gene is first cloned into a pFastBac donor plasmid that contains the left and right part of a Tn7 recombination site and an ampicillin and gentamycin resistance gene. The foreign genetic information is then transferred to a baculovirus shuttle vector (bacmid) via homologous recombination between the Tn7 transposition sites in both pFastBac and the bacmid. The bacmid has a low-copy number mini-F replicon, which enables replication in *E. coli* DHlOBac cells. A LacZ gene is located at the site of insertion, and is disturbed during successful recombination. This enables blue-white screening for positive recombinants. The bacmids also encodes a kanamycin resistance gene and the DHlOBac cells contain a helper plasmid, encoding transposition proteins to support recombination and a tetracycline resistance gene.
Recombined bacmids are then transfected into insect cells, e.g. *Spodoptera frugiperda Sf*9 cells, after which recombinant virus is produced. This virus is subsequently used to generate virus stocks from which SAV VLP expression can be performed (Invitrogen, 2008).

To optimize heterologous protein expression, the P10 gene and the *chitinase and v-cathepsin* genes can be deleted from the viral backbone. P10 is a protein with unknown function which is produced late in infection, in very high amounts. Deletion of P10 increases protein production efficiency. *Chitinase* and *v-cathepsin* are N-glycoproteins, sorted and glycosylated in the ER. They are known for their ability to block the ER and induce ER-stress when produced in high amounts. Deletion of these two genes from the baculovirus expression vector does not affect cell-to-cell infection, but relieves the ER, thereby enabling more efficient processing of ER-targeted recombinant proteins (Slack and Arif, 2007) (Nettleship et al, 2010) (Possee et al, 2008).

Since baculovirus based expression systems are known in the art for several decades by now, further reference is made to standard text books and the information in the Examples section, that will give extensive guidance to the skilled person about how to prepare an immunogenic SAV VLP according to the invention.

Standard text books dedicated to baculovirus based expression systems are i.e.:
Baculovirus and Insect Cell Expression Protocols, by Murahmmer, David W., ISBN: 9781588295378, 2007, Publisher: Springer Verlag.
Baculovirus expression vectors, O'Reilly, D.R., Miller, L.K and Luckow, V.A., ISBN 0195091310, 1994, Oxford Univ. Press.
Baculovirus expression systems are always used at or close to 27°C because that is the optimal temperature for both the infection process and the expression process.

The immunogenic SAV VLP's according to the present invention can now for the first time be made, and they are very suitable as a vaccine component for combating SAV infection; after all, they closely mimic virus particles and as such comparable to inactivated whole viruses.
Therefore, a second embodiment of the present invention relates to an immunogenic SAV VLP according to the invention for use as a medicament.

More specifically, this embodiment relates to an immunogenic SAV VLP according to the invention for use in the treatment or prevention of salmonid fish against SAV infection.

These immunogenic SAV VLP's are made in a baculovirus based expression system and driven by a baculovirus expression vector.

Described herein is a baculovirus expression vector comprising a polynucleotide encoding Alphavirus Capsid Protein, E3 protein, E2 envelope protein, 6K protein and E1 envelope protein, wherein at least the E2 envelope protein and E3 envelope protein is a Salmonid Alphavirus (SAV) E2 envelope protein.

More preferably, not only the E2 envelope protein and E3 envelope protein, but the whole polynucleotide originates from SAV.

Thus, a more preferred baculovirus expression vector is characterised in that it comprises a polynucleotide encoding Salmonid Alphavirus (SAV) Capsid protein, E3 protein, E2 envelope protein, 6K protein and E1 envelope protein.

Still another embodiment of the present invention relates to the use of a baculovirus based expression system for the production of an immunogenic SAV VLP.

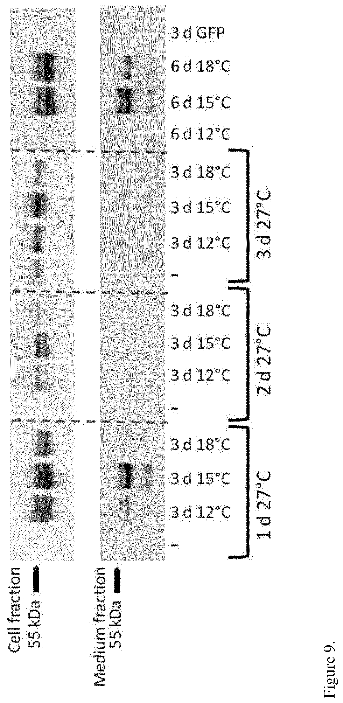

At the lower temperature limit of 12 degrees, baculovirus infection does hardly occur. This can be seen in e.g. figure 9, right column, where infection and maintenance of the cells at 12, 15 and 18 degrees is shown. At 15°C and 18 °C however, a significant level of infection occurs, followed by expression of the E2-protein, and E3 envelope protein. Again, the optimal temperature is in the range from 15-18 degrees.

Thus, another embodiment of the present invention relates to a method for the production of an immunogenic SAV VLP in a baculovirus based expression system, said method comprising the steps of
a) infecting insect cells with a baculovirus expression vector comprising a polynucleotide encoding Alphavirus Capsid protein. E3 protein. E2 envelope protein. 6K protein and E1 envelope protein, wherein at least the E2 envelope protein and E3 envelope protein is a Salmonid Alphavirus (SAV) E2 envelope protein at a temperature of between 12 °C - 22 °C,
b) growing cells at a temperature of between 12 °C - 22 °C and
c) optionally isolating the VLP's from the culture medium

In this method the cells are grown for several days, e.g. 2-10 days, preferably 4-8 days.

Even more surprisingly it was found that immunogenic SAV VLP's can be made in a shorter time and in higher amounts if infection is performed at 27 °C followed by a high-to-low temperature shift after the infection step. A high-to-low temperature shift in practise would mean a shift from 27 °C to a temperature in the range of 12-22 °C, preferably 12-20 °C, more preferably 15-18 °C. The temperature shift may be performed between immediately after the infection step and about a week after the infection step, but is preferably performed between 0 and 100 hours after the infection step. More preferably, the temperature shift is performed between 0 and 24 hours after the infection step.

The infection step comprises the contacting of insect cells with a baculovirus, followed by entry of the virus into the cells. The infection step is considered to take about 4-8 hours at 27 °C.

If the high-to-low temperature shift is performed within 4 hours after infection, this would be considered to be identical to an approach as described in the first method (the three step method) given above. The reason is that in such cases the factual infection step (requiring between 4 and 8 hours) would then take place at a temperature of between 12 °C - 22 °C.

Thus, again another embodiment of the present invention relates to a method for the production of immunogenic SAV VLP in a baculovirus based expression system, said method comprising the steps of
a) infecting insect cells with a baculovirus expression vector comprising a polynucleotide encoding Alphavirus Capsid protein, E3 protein. E2 envelope protein. 6K protein and E1 envelope protein, wherein at least the E2 envelope protein and E3 envelope protein is a Salmonid Alphavirus (SAV) E2 envelope protein at a temperature of between 22 °C - 27°C,
b) lowering the temperature of the cell culture to a temperature of between 12 °C - 22 °C,
c) growing cells at a temperature of between °C - 22 °C and
d) optionally isolating the VLP's from the culture medium

For both methods it is true that the step of growing the cells is preferably done at a temperature of between 15 °C - 18 °C.
Thus, a preferred embodiment for both methods relates to a method for the production of immunogenic SAV VLP in a baculovirus based expression system according to the invention, characterised in that the step of growing the cells is performed at a temperature of between 15 °C -18°C

The temperature shift can in principle be done immediately after infection or one or more days after infection. It was found however, that the temperature shift is preferably made within three days after infection. More preferably, the temperature shift is made within two days after infection, even more preferably within one day after infection. As follows from figure 9, the amount of E2 protein found on cells and in the medium in the form of VLP's reaches the highest level if the temperature shift is performed within one day after infection, when compared to applying a temperature shift only after two or three days.

It was also surprisingly found that the amount of E2 envelope protein, both on cells and on the surface of VLP's in the medium is higher when the cells are submitted to a temperature shift from 27 °C to between 15 °C and 18 °C, than to a shift from 27 °C to 12 °C. It seems that a shift from 27 °C to 12 °C gives a lower amount of E2 envelope protein, which is most likely due to the fact that 12 °C is about the lower temperature limit for insect cells.

Therefore, in a preferred embodiment of the present invention the temperature shift is from 27 °C to a temperature of 15 °C - 18°C.

The Examples section provides ample examples of time spans for growing the cells during the various stages (*vide infra*).

Examples of insect cells that can be used for the expression of VLP's according to the invention are Sf9 cells, Sf21 cells and Trichoplusia ni cells such as Trichoplusia ni Hi Five cells (Invitrogen Corporation, 1600 Faraday Ave. Carlsbad, CA 92008, USA).
Since it is now for the first time possible to make immunogenic SAV VLP's in insect cells, an aspect of the disclosure relates to an insect cell comprising a baculovirus expression vector according to the invention.

As mentioned (*vide supra*), the fact that immunogenic SAV VLP's can now be made means that vaccines for the treatment or prevention of salmonid fish against SAV infection based upon immunogenic SAV VLP's are now for the first time available.
Vaccines for use in the prevention of salmonid fish against SAV infection according to the invention basically comprise an immunogenic amount of immunogenic SAV VLP's and a pharmaceutically acceptable carrier.

Thus, again another embodiment of the present invention relates to vaccines for use in the prevention of salmonid fish against SAV infection, characterised in that such vaccines comprise an immunogenic amount of immunogenic SAV VLP's according to the invention and a pharmaceutically acceptable carrier.

Immunogenic SAV VLP's according to the invention are SAV VLP's that are capable of inducing neutralizing antibodies against SAV in fish. Such immunogenic SAV VLP's according to the invention can be identified by their characteristic that these VLP's react with neutralizing antibodies raised against the SAV E2 envelope protein. Such neutralizing antibodies can e.g. be obtained from fish that recovered from a field infection with wild-type SAV. Preferably they react with neutralizing antibodies raised against a neutralizing immunogenic epitope in the region between amino acid 158 and amino acid 252 of the E2 envelope protein. Patent application WO 2007/031572 describes how to obtain such neutralizing antibodies, and how to perform virus neutralization tests. Moriette, C. et al., describe in general how to obtain monoclonal antibodies against SAV in the Journal of General Virology 86; 3119-3127 (2005).

The term "an immunogenic amount of immunogenic SAV VLP's" as used herein relates to the amount of immunogenic SAV VLP's according to the invention that is necessary to induce an immune response in fish that decreases the pathological effects caused by infection with a wild-type SPDV, when compared to the pathological effects caused by infection with a wild-type SPDV in non-immunized fish.

As said, VLP's closely resemble inactivated whole virus particles. In this respect, protocols for vaccination with inactivated whole salmonid alphavirus, as described e.g. in European Patent EP 0712926, or in the instructions for use that come with a commercially available SAV vaccine such as Norvax PD (Intervet Int. B.V.) thus equally apply to vaccination with immunogenic SAV VLP's.

In principle, the insect cells that produce the VLP's carry the E1/E2 trimeric heterodimers on their surface. (These E1/E2 trimeric heterodimers appear on the surface of the VLP's as a result of the budding of the viral nucleocapsid core through the cell membrane). Therefore, the insect cells as such can also very suitably be used in a vaccine. The amount of E1/E2 trimeric heterodimers per cell can easily be determined and compared to the amount present on VLP's, as indicated in the Examples section. The amount of insect cells necessary for a dose of vaccine would be determined by the amount of E1/E2 trimeric heterodimers per cell. The total amount of E1/E2 trimeric heterodimers per dose would roughly be equal to the amount present in 10³ to 10¹⁰ VLP's (*vide infra*).

Thus, another embodiment relates to a vaccine for the treatment or prevention of salmonid fish against SAV infection, characterised in that said vaccine comprises an immunogenic amount of insect cells directly obtained by applying a method according to the invention and a pharmaceutically acceptable carrier.

In a preferred embodiment of the present invention, a vaccine for use in the prevention of salmonid fish against SAV infection comprises an adjuvant.

The amount of immunogenic SAV VLP's administered will depend (as is the case for whole inactivated SAV) on the route of administration, the presence of an adjuvant and the moment of administration. Generally spoken, vaccines manufactured according to the invention that are based upon VLP's can be administered by injection in general in a dosage of 10³ to 10¹⁰ VLP's, preferably 10⁶ to 10⁹, more preferably between 10⁸ and 10⁹ VLP's. A dose exceeding 10¹⁰ VLP's, although immunologically suitable, will be less attractive for commercial reasons.
For the amount of VLP's in a vaccine dose manufactured according to the invention, the examples below will provide ample guidance.

Examples of pharmaceutically acceptable carriers that are suitable for use in a vaccine for use according to the invention are sterile water, saline, aqueous buffers such as PBS and the like. In addition a vaccine according to the invention may comprise other additives such as adjuvants, stabilizers, anti-oxidants and others, as described below.

Vaccines for use according to the present invention may in a preferred presentation also contain an immunostimulatory substance, a so-called adjuvant. Adjuvants in general comprise substances that boost the immune response of the host in a non-specific manner. A number of different adjuvants are known in the art. Examples of adjuvants frequently used in fish and shellfish farming are muramyldipeptides, lipopolysaccharides, several glucans and glycans and Carbopol^{(R)}. An extensive overview of adjuvants suitable for fish and shellfish vaccines is given in the review paper by Jan Raa (Reviews in Fisheries Science 4(3): 229-288 (1996)).

Oil adjuvants suitable for use in water-in-oil emulsions are e.g. mineral oils or metabolisable oils. Mineral oils are e.g. Bayol^{®}, Marcol^{®} and Drakeol^{®}.
An example of a non-mineral oil adjuvants is e.g. Montanide-ISA-763-A.
Metabolisable oils are e.g. vegetable oils, such as peanut oil and soybean oil, animal oils such as the fish oils squalane and squalene, and tocopherol and its derivatives.
Suitable adjuvants are e.g. w/o emulsions, o/w emulsions and w/o/w double-emulsions An example of a water-based nano-particle adjuvant is e.g. Montanide-IMS-2212.

The vaccine according to the invention may additionally comprise a stabiliser.
A stabilizer can be added to a vaccine according to the invention e.g. to protect it from degradation, to enhance the shelf-life, or to improve freeze-drying efficiency. Useful stabilizers are i.a. SPGA (Bovarnik et al., 1950, J. Bacteriology, vol. 59, p. 509), skimmed milk, gelatine, bovine serum albumin, carbohydrates e.g. sorbitol, mannitol, trehalose, starch, sucrose, dextran or glucose, proteins such as albumin or casein or degradation products thereof, and buffers, such as alkali metal phosphates.

In addition, the vaccine may comprise one or more suitable surface-active compounds or emulsifiers, e.g. Span^{®} or Tween^{®}.

The vaccine may also comprise a so-called "vehicle". A vehicle is a compound to which the polypeptide or the protein according to the invention adheres, without being covalently bound to it. Such vehicles are i.a. bio-microcapsules, micro-alginates, liposomes and macrosols, all known in the art. A special form of such a vehicle is an Iscom, described above.

It goes without saying that admixing other stabilizers, carriers, diluents, emulsions, and the like to vaccines according to the invention are also within the scope of the invention. Such additives are for instance described in well-known handbooks such as: "Remington: the science and practice of pharmacy" (2000, Lippincot, USA, ISBN: 683306472), and: "Veterinary vaccinology" (P. Pastoret et al. ed., 1997, Elsevier, Amsterdam, ISBN: 0444819681).

For reasons of e.g. stability or economy a composition according to the invention may be freeze-dried. In general this will enable prolonged storage at temperatures above zero ° C, e.g. at 4°C. Procedures for freeze-drying are known to persons skilled in the art, and equipment for freeze-drying at different scales is available commercially.
Therefore, the vaccine according to the invention might be in a freeze-dried form.

To reconstitute a freeze-dried composition, it is suspended in a physiologically acceptable diluent. Such a diluent can e.g. be as simple as sterile water, or a physiological salt solution. In a more complex form the freeze-dried vaccine may be suspended in an emulsion e.g. as described in EP 1,140,152.

A vaccine may take any form that is suitable for administration in the context of aqua-culture farming, and that matches the desired route of application and desired effect. Preparation of a vaccine is carried out by means conventional for the skilled person.
Preferably the vaccine is formulated in a form suitable for injection or for immersion vaccination, such as a suspension, solution, dispersion, emulsion, and the like. Commonly such vaccines are prepared sterile.

Target animal for the vaccine according to the invention is a salmonid fish, preferably a rainbow trout (*Oncorhynchus mykiss*) or an Atlantic salmon (*Salmo salar L.*)

Many ways of administration, all known in the art can be applied. The VLP's according to the invention are preferably administered to the fish via injection, immersion, dipping or per oral.

Intraperitoneal application is an attractive way of administration. Especially in the case of intraperitoneal application, the presence of an adjuvant would be preferred, however from an immunological point of view, intraperitoneal vaccination with VLP's is a very effective route of vaccination.

This route of vaccination is however more labor intensive than application routes such as immersion.
Oral and immersion vaccination routes are preferred for ease of administration of the vaccine.

For oral administration the vaccine is preferably mixed with a suitable carrier for oral administration i.e. cellulose, food or a metabolisable substance such as alpha-cellulose or different oils of vegetable or animals origin. Also an attractive method is administration of the vaccine to high concentrations of live-feed organisms, followed by feeding the live-feed organisms to the fish. Particularly preferred food carriers for oral delivery of the vaccine according to the invention are live-feed organisms which are able to encapsulate the vaccine. Suitable live-feed organisms include plankton-like non-selective filter feeders, preferably members of Rotifera, *Artemia,* and the like. Highly preferred is the brine shrimp *Artemia* sp.

The age of the fish to be vaccinated is not critical, although clearly one would want to vaccinate fish against SAV infection in as early a stage as possible, i.e. prior to possible exposure to the pathogen.
Immersion vaccination would be the vaccination of choice especially when fish are still small, e.g. between 2 and 5 grams. Fish from 5 grams and up can, if necessary or desired, also be vaccinated by means of injection.

Otherwise, man skilled in the art finds sufficient guidance in the references mentioned above and in the information given below, especially in the Examples.

Review articles relating to fish vaccines and their manufacture are i.a. by Sommerset, I., Krossøy, B., Biering, E. and Frost, P. in Expert Review of Vaccines 4: 89-101 (2005), by Buchmann, K., Lindenstrøm, T. and Bresciani, in J. Acta Parasitologica 46: 71-81 (2001), by Vinitnantharat, S., Gravningen, K. and Greger, E. in Advances in veterinary medicine 41: 539-550 (1999) and by Anderson, D.P. in Developments in Biological Standardization 90: 257-265 (1997). Moreover, the skilled practitioner will find guidance in the Examples below.

It would be beneficial to combine immunogenic SAV VLP's with at least one other vaccine against a fish-pathogenic microorganism or virus.

The combined use of immunogenic SAV VLP's and at least one fish-pathogenic bacterium or virus for the manufacture of the vaccine would have the benefit that protection is obtained against both bacterial infection and infection with said fish-pathogenic virus.

Therefore, a preferred form of this embodiment relates to a combination vaccine for use in the prevention of salmonid fish against SAV infection, characterised in that said vaccine comprises immunogenic SAV VLP's according to the invention and at least one other vaccine against a fish-pathogenic microorganism or a fish-pathogenic virus.

Examples of commercially important cold water fish pathogens are *Vibrio anguillarum, Aeromonas salmonicidae, Vibrio salmonicidae, Moritella viscosa, Vibrio ordalii, Flavobacterium sp., Flexibacter sp., Streptococcus sp., Lactococcus garvieae, Edwardsiella tarda, E. ictaluri, Piscirickettsia salmonis,* Viral Nervous Necrosis virus, Infectious Pancreatic Necrosis virus and iridovirus.

Therefore, preferably, the said fish-pathogenic microorganism or a fish-pathogenic virus would be selected from the group consisting of *Vibrio anguillarum, Aeromonas salmonicidae, Vibrio salmonicidae, Moritella viscosa, Vibrio ordalii, Flavobacterium sp., Flexibacter sp., Streptococcus sp., Lactococcus garvieae, Edwardsiella tarda, E. ictaluri, Piscirickettsia salmonis,* Viral Nervous Necrosis virus, Infectious Pancreatic Necrosis virus and iridovirus.

### EXAMPLES:

### Example 1

### Cells and viruses.

Adherent *Spodoptera frugiperda* (*Sf9*) cells (Invitrogen) were maintained as monolayer cultures in Sf-900 II medium (Gibco), supplemented with 5% foetal calf serum. The Bac-to-Bac recombinant baculovirus based expression system (Invitrogen) was used to generate recombinant baculoviruses using a modified *Autographa californica* multicapsid nucleopolyhedrovirus (AcMNPV) backbone. Promoter sequences and large parts of the coding sequences of cathepsin and chitinase were deleted from the bacmid backbone (Kaba, 2004). The resulting AcΔcc was further modified by deletion of the p10 promoter and ORF. These elements were replaced by a zeocin resistance marker by Lambda Red recombination (Datsenko, 2000)(Pijlman, 2002). Modified LoxP sequences flanking the zeocin resistance marker were used for subsequent removal of the resistance marker by Cre recombinase (Suzuki, 2005). Ac-GFP (Pijlman, 2001) was used as a control in some experiments. Recombinant baculovirus titres expressed as tissue culture infectious dose 50 (TCID₅₀) per ml, were determined by end point dilution. All infections were performed in serum free Sf-900 II medium.

### Construction of the recombinant baculovirus expressing the SAV structural cassette.

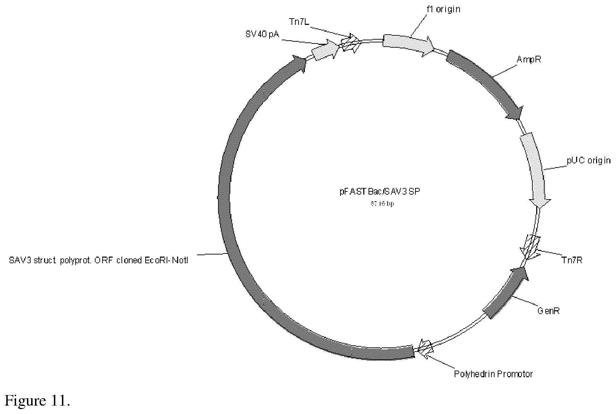

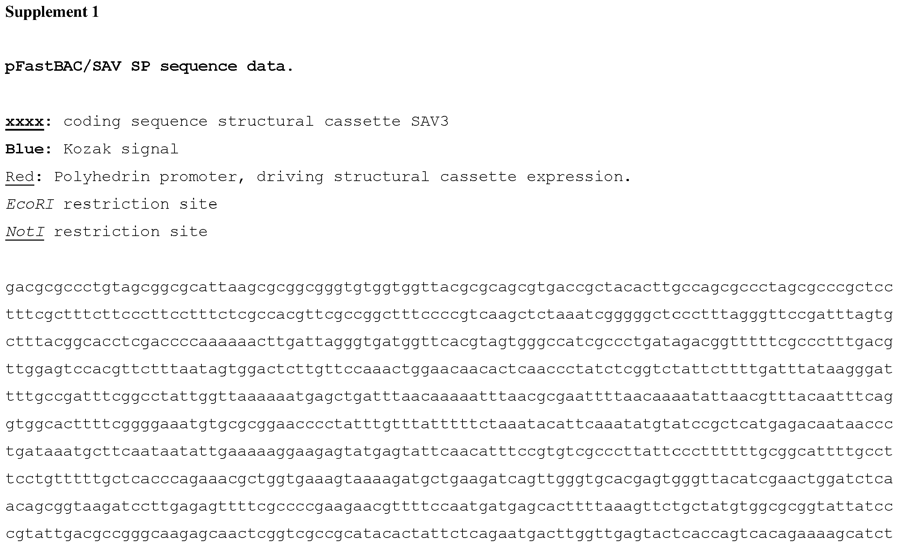

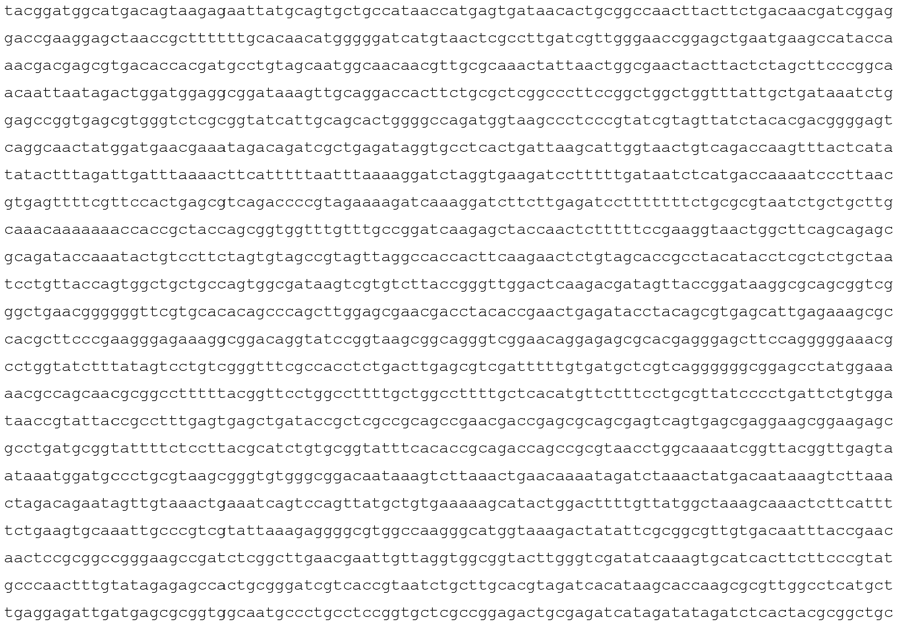

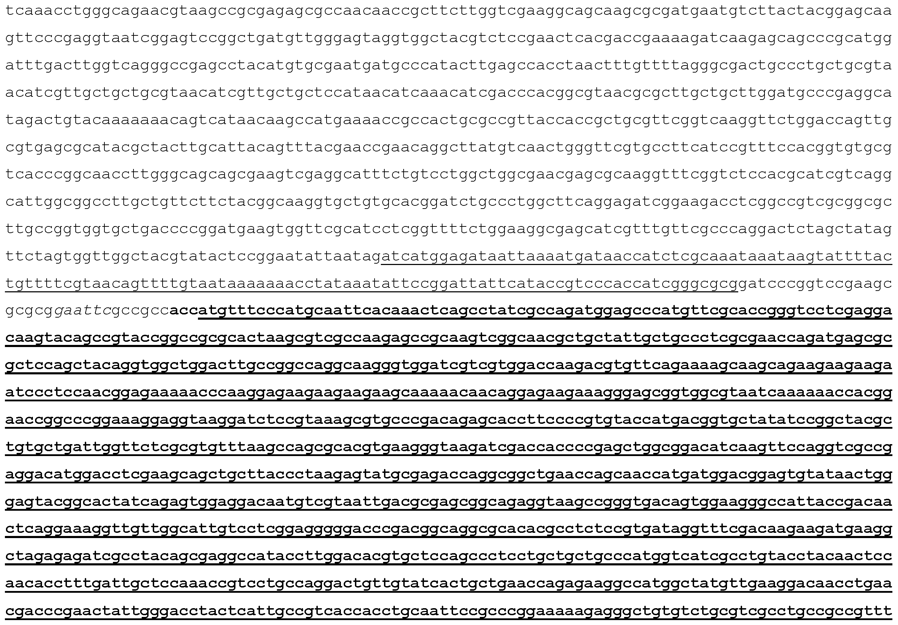

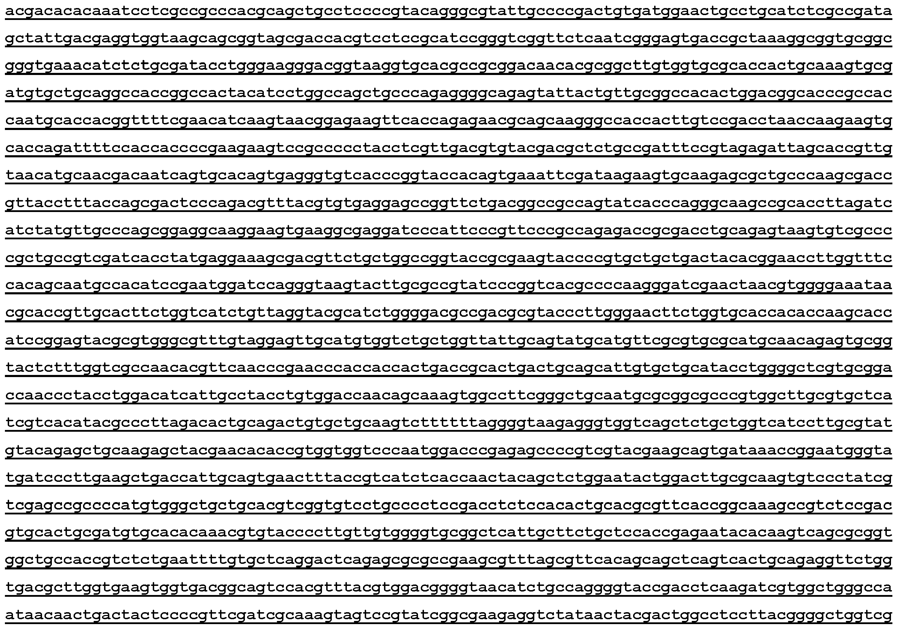

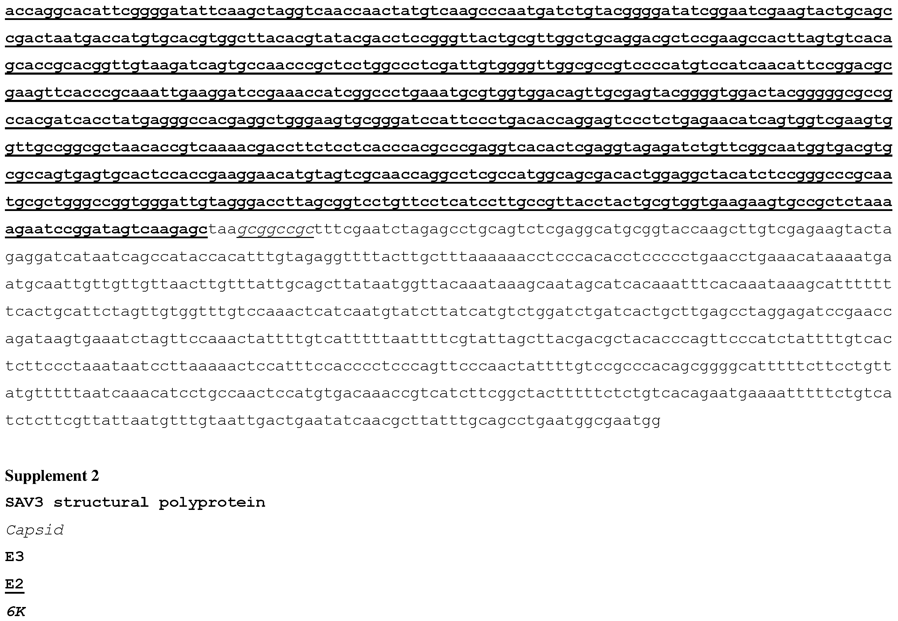

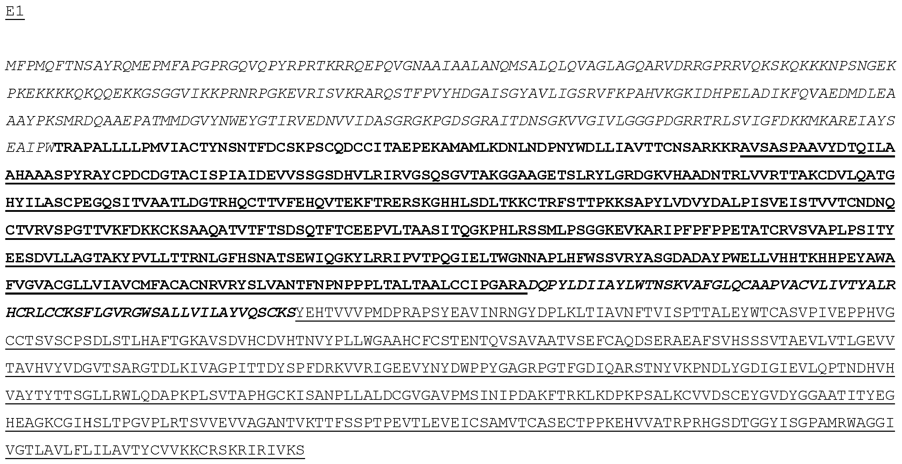

The SAV structural cassette was cloned as an EcoRI-NotI restriction fragment into pFastBAC1 (Invitrogen), generating pFastBac1 / SAV. The structure of pFastBAC / SAV3 is presented in figure 11. Sequence data of pFastBAC / SAV3 and of the SAV3 structural proteins are provided in Supplement 1 and 2. The recombinant baculovirus AcΔccΔp10 expressing the structural cassette of SAV was generated using the Bac-to-Bac baculovirus based expression system, resulting in Ac-structural.

### Recombinant baculovirus infections and temperature shift assay.

To analyze protein expression and processing, 1.5x10⁶ *Sf*9-cells were seeded in a 6-wells culture plate and infected with Ac-structural at a multiplicity of infection (MOI) of 10 TCID₅₀ units per cell. Infections were performed at 27°C for 4 h on a shaking platform. The virus suspension was replaced with fresh medium. Infected cells were incubated at 12°C, 15°C, 18°C or 27°C for respectively 14 days, 10 days, 7 days and 3 days. The medium fraction was removed by centrifugation and cells were harvested, washed once in phosphate buffered saline (PBS), resuspended in 200µl PBS and stored at-20°C. To analyze the effect of a temperature-shift on protein processing, cells were infected and harvested as previously described, but were initially incubated at 27°C. Next, cells were transferred to 12°C, 15°C or 18°C at 24 hpi, 48 hpi or 72 hpi.

### Recombinant baculovirus-infections at 12°C.

To investigate if recombinant baculoviruses are able to infect insect cells at 12°C, 3.0x10⁶ *Sf*9-cells were seeded in a 25cm² culture flask and were infected with a recombinant AcMNPV virus, expressing polyhedrin and GFP under control of the P10 promoter (Ac-polh-GFP). GFP expression and polyhedra formation was used to monitor virus replication. In order to maintain the temperature during infection, T25 flasks were kept on icepacks preincubated at 12°C. To keep cells at a stable 12°C until microscopic analysis at 2, 4, 6 and 8 dpi at room temperature, 4 culture flasks were seeded in parallel and infected at 12°C.

### Protein analysis.

Secreted protein fractions were precipitated by 7% NaCl₂, 2.3%(w/v) polyethylene glycol(PEG)-6000 precipitation or by filtration through a 10K ultracel centrifugal filter (Amicon). Precipitation pellets were resuspended in 100µl PBS and stored at -20°C. Whole cell lysates and medium fractions were analyzed by sodium dodecyl sulphate polyacrylamide gel electrophoresis (SDS-PAGE) and Coomassie brilliant blue (CBB) staining. Denatured proteins were transferred to an Immobilon membrane (Millipore) for Western blot (WB) analysis. Membranes were blocked with 3% skim milk in PBS-0.1% Tween-60 (PBST) for 1 hr at room temperature (RT). Membranes were washed in PBST and incubated for 1 h at RT with 1:2000 diluted primary monoclonal antibody mouse α-E2 (SAV) in PBST, supplemented with 0.2% skim milk. Membranes were washed 3 times with PBST incubated for 45 min at RT with 1:3000 diluted Alkaline Phosphatase (AP) conjugated goat α-mouse, in PBST supplemented with 0.2% skim milk. Membranes were washed three times in PBST and incubated with AP-buffer for 10 minutes. Proteins were detected by NBT/BCIP staining (Roche).

### PNGase F treatment.

Protein fractions were treated with PNGase F (New England Biolabs) to analyze the glysosylation status of SAV-E2. Cell fractions and the precipitated medium fraction were treated with 1µl glycoprotein denaturing buffer in 9µl MilliQ for 10 min at 100°C. The denatured protein-mix was incubated for 1 h at 37°C with 2 µl 10x G7 reaction buffer, 2 µl 10% NP40, 1.5 µl PNGase F and 4.5 µl MilliQ. Treated proteins fractions were analyzed with SDS-PAGE, WB and CBB.

### Immunofluorescence assay.

*Sf*9 cells were seeded on 13 mm glass coverslips and infected as described under **Recombinant baculovirus infections and temperature shift assay** (*vide supra*) and incubated at 12°C, 15°C, 18°C and 27°C. Cells were fixed with 4% paraformaldehyde for 5 minutes at RT, washed with PBS and subsequently incubated with PBS containing 1:2000 diluted primary monoclonal antibody mouse α-E2 (SAV) for 1hr at RT. This monoclonal is a neutralising α-E2 monoclonal, obtained as described in WO 2007/031572.
Cells were washed carefully with PBS and treated with 1:1000 diluted goat α-mouse polyclonal Alexa 546 labeled antibody (Invitrogen) for 1hr at RT in dark conditions. Next, cells were washed and coverslips were loaded onto an object glass with a drop of antifade fluoromount-G (Southern Biotech). Cells were analyzed by laser confocal microscopy on a Zeiss LSM 510 Meta, Axiovert 100m.

### Electron microscopy analysis.

Copper 400 square mesh grids (Veco) were hydrophilized by Argon gas decharge. Next, 10 µl VLP sample was loaded onto a grid for 2 minutes and carefully removed. The grid was washed 5x with MilliQ and stained with 2% uranyl acetate for 20 s. Excess dye was removed, grids were air dried and analyzed with a JEOL JEM1011 transmission electron microscope (2007) and iTEM Soft imaging system (Olympus) software.

### RESULTS

### Expression of SAV structural cassette in insect cells by recombinant baculoviruses.

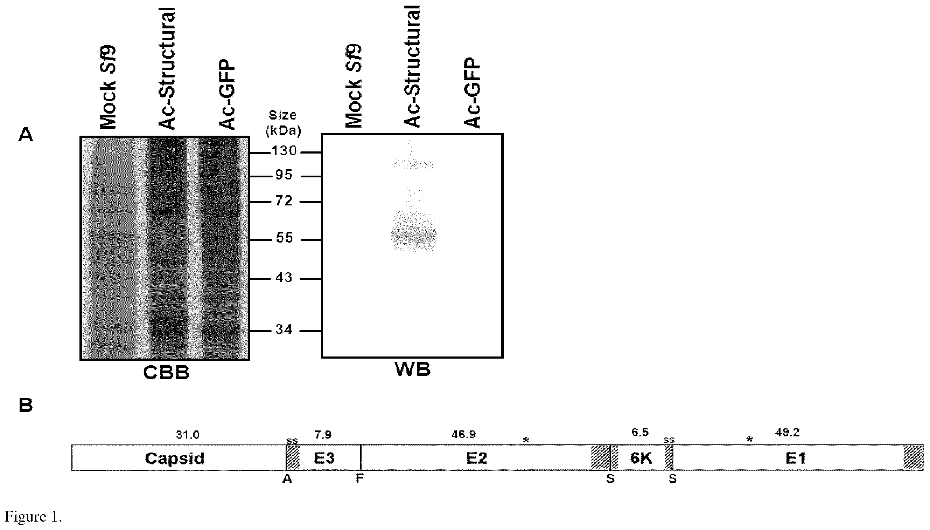

A recombinant baculovirus was generated (Ac-structural) to express the complete SAV structural cassette C-E3-E2-6K-E1 **(Fig. IB).** The complete coding sequence of the structural cassette was cloned downstream of the polyhedrin promoter in a pFastBAC1 vector and then transferred to an adapted AcMNPV backbone (see Material and Methods). A stock of recombinant baculovirus was generated upon transfection of Sf9 cells with purified baculovirus bacmid DNA. Next, Sf9 insect cells were infected with a MOI of 10 and were incubated at 27°C for 72 h. Protein expression in the cell fraction was analyzed by CBB and western analysis using α-E2 monoclonal antibodies. Total protein-staining by CBB showed high levels of expression based upon the protein band of approximately 35kDa, which closely matches the predicted size of the SAV capsid protein **(****Fig.1A****, lane 2).** Western analysis using α-E2 mabs yields a proteins band of approximately 55 kDa **(****Fig.1A****, lane 5).** The size corresponds to the predicted molecular mass of E3E2 (55kDa), thereby indicating that expression of the SAV structural cassette at 27°C results in production of a E3E2 precursor that is not processed by host furin-like proteases into E3 and E2.

### N-linked glycosylation of recombinant SAV-E2.

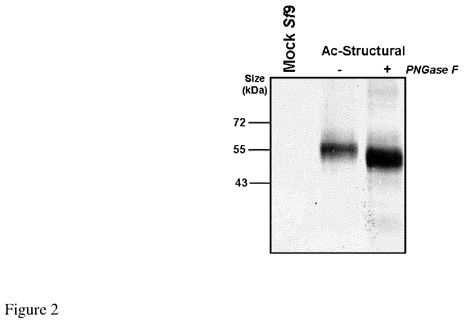

To analyze if the E3E2 fraction found after the expression of the SAV-structural cassette is glycosylated **(****Fig.1A****, lane 5),** expression products were treated with PNGase F (New England Biolabs), which enzymatically removes carbohydrate residues from proteins. The number of glycosylation sites varies, but in general, all alphavirus glycoproteins E1 and E2 are N-linked glycosylated. SAV-E2 is predicted to be glycosylated at a single site, N318 **(****Fig. 1B****)**(Strauss & Strauss, 1994. Weston, 1999). Total cell fractions of infected cells were treated with PNGase F and the effect was visualized by Western blotting **(****Fig.2****).** Analysis of the untreated protein sample results in a protein band with a molecular mass of approximately 55kDa **(****Fig.2****, lane 2).** However, PNGase F treatment on the same cell lysate, yields a protein band with a slightly lower molecular weight **(****Fig.2****, lane 3).** The shift in molecular mass of the SAV-E3E2 fraction after PNGase F treatment suggests that SAV-E2 is indeed N-glycosylated, when expressed in *Sf*9 insect cell by recombinant baculoviruses. The absence of a non-glycosylated protein fraction in the untreated sample indicates that SAV-E2 is efficiently glycosylated in insect cells. However, E3 is not released from E3E2 by furin-processing.

### Temperature-dependent processing of SAV structural proteins.

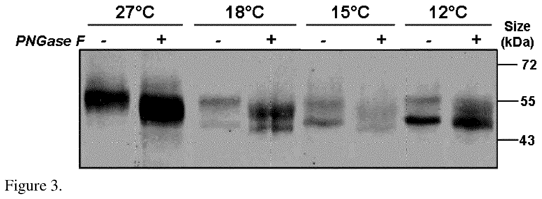

The analysis of the SAV structural cassette, expressed by recombinant baculoviruses at 27°C, showed that SAV-E2 is efficiently glycosylated, but is not released from its E3E2 precursor by host furin-like proteases. The lack of furin-processing in E3E2 might be caused by the biology of SAV replication. In fish cells, SAV is produced at 10-15°C (McLoughlin, 2007) whereas baculovirus expression generally takes place at approximately 27°C. To investigate a putative effect of temperature on furin-dependent processing of E3E2, *Sf*9-cells were infected with Ac-structural and incubated at 12, 15, 18 and 27°C for 14, 10, 7 and 3 days, respectively. Whole cell lysates were treated with PNGase F and analyzed using western blot. Expression at 27°C **(****Fig.3****, lane 1-2)** resulted in similar protein patterns as seen before **(****Fig.2****, lane 2-3).** The 55kDa protein band was found at all other lower expression temperatures **(****Fig.3****, lane 3, 5, 7),** but a second protein band with lower molecular mass was observed. The smaller polypetides (48kDa) closely resemble processed SAV-E2 by predicted molecular mass (46.9kDa). This suggests that expression of Ac-structural at 18°C, 15°C and 12°C in insect cells enables furin-dependent processing, yielding SAV-E2. Furthermore, PNGase F treatment shows a shift in molecular mass of both predicted E3E2 and E2 at all expression temperatures **(****Fig.3****, lane 4, 6, 8),** indicating that E3E2 as well as E2 is N-glycosylated. Significantly, the relative intensity of E2 increased with a decrease of the temperature, which suggests that SAV-E2 processing is temperature dependent.

### Surface expression of baculovirus expressed SAV-E2 in insect cells.

To generate progeny viral particles through budding, alphavirus glycoproteins assemble into heterodimers. Three of these E1-E2 heterodimers congregate into heterotrimers and form the so-called trimeric spikes that are exposed at the surface of the virion. The spikes are formed in the ER and pass through the Golgi apparatus. At the end of the processing pathway, the trimeric spikes are anchored in the plasma membrane by the C-terminal transmembrane domains of E1 and E2 and thus are expressed on the surface of infected cells. So far, it has been shown that expression of the SAV structural cassette by recombinant baculoviruses in insect cells results in the formation of N-glycosylated E3E2, which, at lower temperatures, is processed to glycosylated E2. This might suggest that processing in insect cells resembles the processing during normal SAV infections. To analyze whether SAV glycoprotein spikes are expressed at the cell surface of Sf9 cells, an infection with Ac-structural was carried out at different temperatures. Next, cells were subjected to immunofluorescence using α-E2 mabs **(****Fig.4****).** Positive staining indicates that SAV-E2 is exposed at the surface of the cells. Confocal microscopy reveals that the detection of SAV-E2 at the plasma membrane of insect cells is again temperature dependent. The ring-like staining, which indicates surface expression of E2, can only be found when cells infected with Ac-structural were placed at 12°C, 15°C and 18°C **(****Fig.4****, middle).** Expression at 27°C results in negatively stained cells **(****Fig.4****, top),** resembling the mock cell control **(****Fig.4****, bottom).** This data complies with the previously found results, and suggests that the processing and surface expression of SAV-E2 is temperature dependent.

### SAV virus-like particle production in insect cells by recombinant baculoviruses expressing the SAV structural cassette.

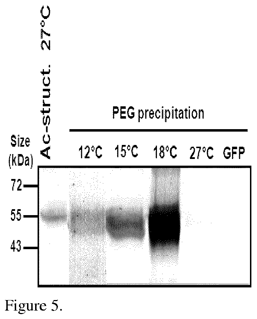

This study has shown that the processing and surface expression of N-glycosylated SAV-E2, expressed from the SAV structural cassette by recombinant baculoviruses in insect cells, is temperature dependent. Mature, N-glycosylated SAV-E2 is produced at temperatures ranging from 12°C to 22°C and is expressed at the surface of Ac-structural infected cells. In addition, it has been shown that the capsid protein is successfully auto-cleaved from the structural polyprotein **(****Fig.1A****, lane 2).** To investigate if free capsid and surface expressed glycoproteins are able to form virus-like particles (VLP), the medium fraction of Ac-structural infected *Sf*9 cells was concentrated by PEG-precipitation. The presence of SAV-E2 served as an indicator for VLP formation. Western analysis using α-E2 mabs showed that E2 could be detected in the medium fraction, when proteins were expressed at 12°C, 15°C and 18°C **(****Fig.5****, lane 2-4).** In sharp contrast, when the structural cassette was expressed at 27°C, E2 was retained in the cell fraction as E3E2 precursor **(****Fig.5****, lane 1, 5).** The quantity of E2 found in the medium fraction increased at higher temperatures, which probably relates to higher overall expression levels..

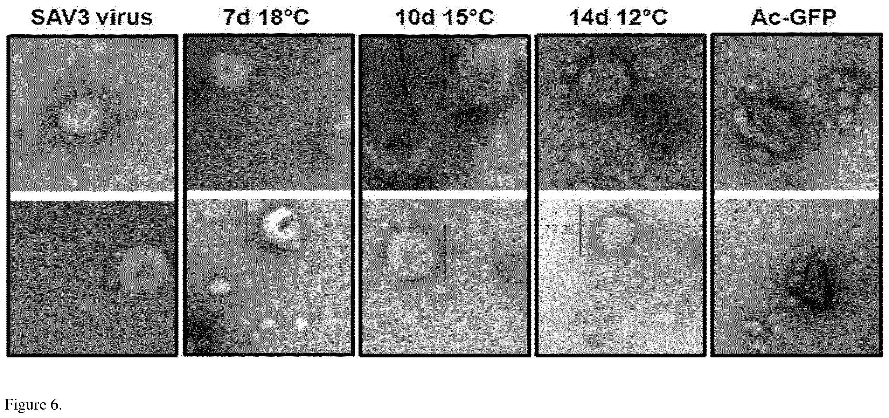

The fact that SAV-E2 was detected in the medium fraction of infected cells, suggested that SAV virus-like particles were formed. To further investigate VLP formation at different temperatures, medium fractions were analyzed using electron microscopy (EM). Non-precipitated medium of an Ac-structural infected *Sf*9 cell culture was loaded onto copper-grids and stained **(****Fig.6****).** VLP-like structures - spherical particles of 65-70 nm - were found when the SAV structural cassette was expressed at 12°C, 15°C and 18°C, but were absent in the control Ac-GFP infection group **(****Fig.6****).**

### Recombinant baculovirus infection at 27°C versus 12°C in insect cells.

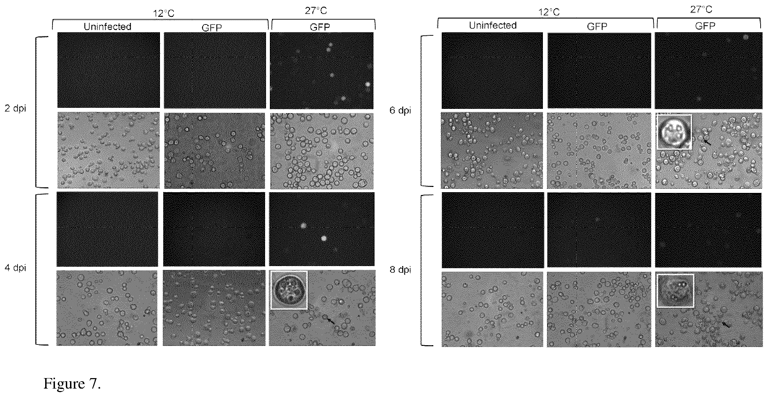

This study has shown that processing and maturation of SAV-E2 is a temperature dependent process. Production of SAV structural proteins in *Sf*9 cells at 27°C has been shown to be disadvantageous for E2 processing and surface expression. In the experiments carried out at 12°C, at which temperature the furin-dependent cleavage of the E3E2 precursor is most efficient, the baculovirus infection was executed at 27°C for 4 h. After that, the infected cells were transferred to 12°C and were incubated for 14 days. To see if primary baculovirus infection has to be carried out at 27°C, or whether baculoviruses are able to infect cells at 12°C, *Sf*9 cells were infected at strict temperature conditions of 27°C and 12°C with a recombinant baculovirus expressing polyhedrin and GFP under control of the polyhedrin- and P10-promoters, respectively. Formation of polyhedral and expression of GFP were used as markers for infection. GFP expression was detected 2 days post infection (dpi) and polyhedra formation was observed 4 dpi, in cells infected and incubated at 27°C **(****Fig.7****, lane 3,6).** Neither GFP expression or polyhedra formation was observed 2, 4 or 6 dpi at cells infected and incubated at 12°C **(****Fig.7****, lane 2,5).** However, a weak GFP signal (but no polyhedra formation) was detected 8 dpi at cells infected and incubated at 12°C **(****Fig.7****, lane 5).** These results indicate that baculovirus infection at 12 °C is very inefficient and does not lead to polyhedra formation within a week.

### SAV structural cassette expression and VLP formation by temperature-shift in insect cells.

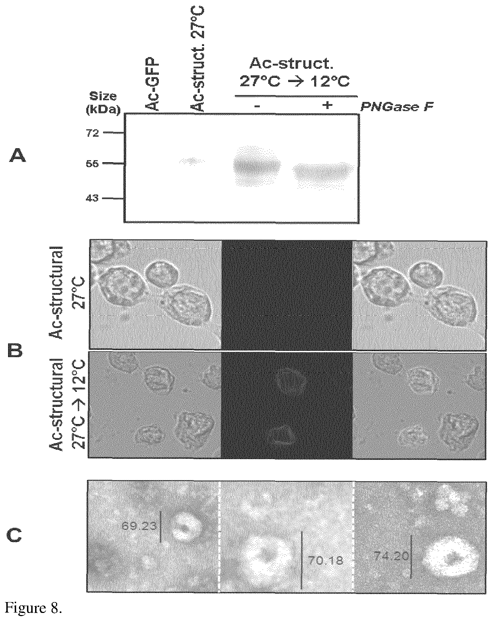

It was found that recombinant baculoviruses may be able to infect *Sf*9 insect cells at 12°C. However, infection at 12°C develops significantly less efficient than infection performed at 27°C **(****Fig.7****).** Interestingly, previous results have shown that the opposite is true for SAV glycoprotein processing and secretion. To optimize both the baculovirus infection and SAV structural polyprotein processing, a temperature-shift experiment was performed. *Sf*9 cells were infected with Ac-structural at 27°C for 2 days and cells were transferred to 12°C for a further 3 days. Protein expression and processing was analyzed 5dpi by western blot **(****Fig.8A****).** The temperature-shift resulted in increased processing of SAV-E3E2 **(****Fig8A****, lane 4),** since both SAV-E3E2 (55kDa) and mature SAV-E2 (48kDa) were detected. PNGase F treatment showed that both E2 configurations were N-glycosylated **(****Fig.8A****, lane 4).** Immunostaining on *Sf*9 cells that were infected following the temperature-shift principle, showed that SAV-E2 was detected on the surface of infected cells **(****Fig.8B****, bottom),** similar to results found after 12°C incubation **(****Fig.4****).** In contrast, cells infected and incubated at 27°C did not show surface expression of E2 **(**Fig.4B**).** In addition, medium of the infected *Sf*9 cell culture was examined by electron microscopy **(****Fig8C****).** VLP-like structures were detected that were similar to those found after strict infection-regimes **(****Fig.6****).**

Previous results have shown that protein processing efficiency is not only enhanced at 12°C, but also at incubation temperatures of 15°C and 18°C. Furthermore, incubation at 27°C ensures proper baculovirus infections but prevents SAV-E2 processing, surface expression and detection in the medium fraction. Therefore, *Sf*9 cells were infected at 27°C and transferred to 12°C, 15°C and 18°C at 1 dpi, 2 dpi and 3 dpi. Cells were subsequently incubated for a further 3 days at the indicated temperatures. For comparison, cells were infected and incubated for 6 days at 12°C, 15°C and 18°C. Both cell and PEG-precipitated medium fractions were analyzed by western analysis using α-E2 mabs **(****Fig.9****).** When cells were infected at 27°C for only 1 day, SAV-E2 was not detected in both cell and medium fraction. **(****Fig.9****, lane 1).** However, when cells were subsequently incubated for three days at lower temperatures, processed E2 was abundantly present in the cell and medium fractions **(****Fig.9****, lane 2-4).** Cells that were infected at 27°C for 2 days, produced a little amount of E3E2. The temperature-shift to lower temperatures at 2 dpi increased processing and detection of E2 in both the cell and medium fraction **(****Fig.9****, lane 5-8),** although it was less than observed with the shift after 1 dpi. In this experiment, a shift to 15°C gave rise to higher expression levels than observed at 12°C and 18°C **(****Fig.9****, lane 7).** Infection for 3 days at 27°C appeared to be highly disadvantageous for E3E2 processing and E2 secretion, since no mature SAV-E2 was detected in both cell and medium fractions **(****Fig.9****, lane 9-12).**

In the cells incubated for 6 days at 12, 15 or 18°C, optimal E2 expression and secretion was observed at 15°C **(****Fig.9****, lane 14),** but expression levels were not as high as with the temperature shift after 1 day **(****Fig.9****, lane 3).** In conclusion, compared to a 6 day 15°C production regime **(****Fig.9****, lane 14),** the 1 day 27°C to 15°C 3 day shift increases total E2 expression/secretion and significantly shortens the total production time from 6 days to 4 days.

### Legend to the figures.

Figure 1: SAV structural cassette expression using recombinant baculoviruses. A) Gene expression in *Sf*9 cells was analyzed by Coomassie brilliant blue (CBB) and western blotting (WB) using α-E2 mabs. B) Schematic representation of the SAV structural cassette as it is expressed by recombinant baculoviruses downstream of the polyhedrin promoter. The molecular mass of the proteins is indicated and shaded areas represent transmembrane domains or signal peptides (ss). A, F and S indicate autocatalytic, furin and signalase cleavage sites, asterisks represent predicted N-linked glycosylation sites.
Figure 2: N-linked glycosylation of SAV-E3E2. Whole cell-lysates were treated with PNGase F and analyzed by western blotting using α-E2 mabs.
Figure 3: Temperature dependent processing of the SAV structural cassette, expressed by recombinant baculoviruses in insect cells. Cells were infected at 12°C, 15°C, 18°C and 27°C and harvested at 14 dpi, 10 dpi, 7 dpi and 3 dpi, respectively. Whole cell lysates were treated with PNGase F and analyzed by western blotting using α-E2 mabs.
Figure 4: SAV3-E2 detection on the surface of *Sf*9 cells after recombinant baculovirus expression. Cells were infected with Ac-structural at 12°C, 15°C, 18°C and 27°C. Cells were fixed with 4% paraformaldehyde but not permeabilized, and subjected to immunostaining with α-E2 mabs. Cells were analyzed by confocal microscopy. Positive staining reveals the presence of E2 at the surface of infected cells.
Figure 5: SAV-E2 detection in the medium fraction of Ac-structural infected *Sf*9 cells. The medium fraction of infected cells was PEG-precipitated. Western blotting using α-E2 mabs reveals the presence of SAV-E2 in the medium at different temperatures.
Figure 6: SAV3-VLP formation in Ac-structural infected *Sf*9 cells at 12°C, 15°C and 18°C. Non-precipitated medium was analyzed by electron microscopy and VLP-like structures were visualized by negative staining.
Figure 7: Recombinant baculovirus infection at 27°c and 12°C. Sf9 cells were infected with AcMNPV expressing polyhedrin and GFP under control of the polyhedrin and P10 promoter, respectively. Polyhedra formation en GFP expression was used as a marker for baculovirus infection.
Figure 8: Temperature-shift assay of SAV structural protein expression by recombinant baculoviruses. Sf9 cells were infected at 27°C for 2 days with Ac-structural and incubated for 3 days at 12°C. A) Whole cell lysates were analyzed by western blotting using α-E2 mabs. B) Cells were subjected to immunostaining using the α-E2 mabs for surface detection of SAV-E2 in infected cells. C) The presence of VLP-like structures was visualized by electron microscopy.
Figure 9: Comparative temperature-shift assay on cells expressing the SAV structural polyprotein by recombinant baculoviruses. Cells were infected with Ac-structural at 27°C for 1 day, 2 days and 3 days. Next, cells were transferred for 3 days to 12°C, 15°C or 18°C. Whole cell lysates and PEG-precipitated medium fractions were analyzed by western blotting using α-E2 mabs
Figure 10: Bac-to-bac baculovirus based expression system from Invitrogen. A pFastBac donor plasmid is used to introduce the gene of interest in the baculovirus vector (bacmid), which can be amplified in *E*. *coli* cells. After isolation of the bacmids, insect cells can be transfected with the recombinant bacmid DNA and recombinant proteins will be produced (Invitrogen, 2008).
Figure 11: Schematic representation of pFastBAC1/SAV. By Vector NTI (Invitrogen)

### Literature.

Datsenko, K. A. & Wanner, B. L. (2000). One-step inactivation of chromosomal genes in Escherichia coli K-12 using PCR products. Proceedings of the National Academy of Sciences of the United States of America 97, 6640-6645.
Hodneland, K., Bratland, A., Christie, K. E., Endresen, C., and Nylund,A. (2005). New subtype of salmonid alphavirus (SAV), Togaviridae, from Atlantic salmon Salmo salar and rainbow trout Oncorhynchus mykiss in Norway. Diseases of Aquatic Organisms 66, 113-120.
Kaba, S. A., Salcedo, A. M., Wafula, P. O., Vlak, J. M. & van Oers, M. M. (2004). Development of a chitinase and v-cathepsin negative bacmid for improved integrity of secreted recombinant proteins. Journal of virological methods 122, 113-118.
Kuhn, R.J., (2007). Togaviridae: The Viruses and Their Replication. Fields Virology (5th edition) Chapter 30: Lippincott Williams & Wilkins. Philadelphia.
Luckow, V.A., Lee, S.C., Bary, G.F., Olins, P.O. (1993) Efficient Generation of Infectious Recombinant Baculoviruses by Site-Specific Transposon-Mediated Insertion of Foreign Genes into Baculovirus Genome Propagated in Escherichia coli. Journal of Virology 67, 4566-4579.
McLoughlin, M. F., and Graham, D. A. (2007). Alphavirus infections in salmonids- a review. Journal of Fish Diseases 30, 511-531.
Nettleship, J.E., Assenberg, R., Diprose, J.M., Rahman-Huq, N., Owens, R.J. (2010). Recent advances in the production of proteins in insect and mammalian cells. Journal of Structural Biology 172, 55-65.
Pijlman, G.P., Van der Bord, E., Martens, D.E., Vlak, J.M. (2001). Autographa californica Baculoviruses with Large Genomic Deletions Are Rapidly Generated in Infected Insect Cells. Virology 283, 132-138.
Pijlman, G. P., Dortmans, J. C., Vermeesch, A. M., Yang, K., Martens, D. E., Goldbach, R. W. & Vlak, J. M. (2002). Pivotal role of the non-hr origin of DNA replication in the genesis of defective interfering baculoviruses. Journal of virology 76, 5605-5611.
Possee, R.D., Hitchman, R.B., Richards, K.S., Mann, S.G., Siaterij, E., Nixon, C.P., Irving, H., Assenberg, R., Alderton, D., Owens, R.J., King, L.A. (2008) Generation of baculovirus vectors for the high-throughput production of proteins in insect cells. Biotechnology and bioengineering 15, 1115-1122.
Powers, A. M. & Logue, C. H. (2007). Changing patterns of chikungunya virus: re-emergence of a zoonotic arbovirus. The Journal of general virology 88, 2363-2377.
Slack, J., Arif, B.M. (2007). The baculoviruses occlusion-derived virus: virion structure and function. Advances in virus research 69, 99-165.
Strauss, J. H., and Strauss, E. G. (1994). The Alphaviruses: Gene Expression, Replication, and Evolution. Microbiological Reviews 58, 491-562.
Suzuki, N., Nonaka, H., Tsuge, Y., Inui, M. & Yukawa, H. (2005). New multiple-deletion method for the Corynebacterium glutamicum genome, using a mutant lox sequence. Applied and environmental microbiology 71, 8472-8480.
Villoing, S., Arzotti, M., Chilmonczyk, S., Castric, J., Bremont, M. (2000). Rainbow Trout Sleeping Disease Virus Is an Atypical Alphavirus. Journal of Virology 74, 173-183.
Weston, J. H., Welsh, M. D., McLoughlin, M. F. & Todd, D. (1999). Salmon pancreas disease virus, an alphavirus infecting farmed Atlantic salmon, Salmo salar L. Virology 256, 188-195.
Weston, J., Villoing, S., Bremont, M., Castric, J., Pfeffer, M., Jewhurst, V., McLoughlin, M., Rodseth, O., Christie, K. E., Koumans, J. & Todd, D. (2002). Comparison of two aquatic alphaviruses, salmon pancreas disease virus and sleeping disease virus, by using genome sequence analysis, monoclonal reactivity, and cross-infection. Journal of virology 76, 6155-6163.
Weston, J. H., Graham, D. A., Branson, E., Rowley, H. M., Walker, I. W., Jewhurst, V. A., Jewhurst, H. L. & Todd, D. (2005). Nucleotide sequence variation in salmonid alphaviruses from outbreaks of salmon pancreas disease and sleeping disease. Dis Aquαt Organ 66, 105-111.

## Claims

1. Use of a baculovirus expression vector in a method for the production of an immunogenic SAV VLP in a baculovirusbased expression system,
**characterised in that** said expression vector comprises at least genetic information encoding an Alphavirus Capsid protein, E3 envelope protein, and an E2 envelope protein, 6K protein and E1 envelope protein,
wherein at least the E2 and the E3 proteins are a Salmonid Alphavirus (SAV) E2 and E3 envelope proteins and
wherein the method for producing the immunogenic SAV VLP comprises:
(a) Infecting insect cells with the said baculovirus expression vector either
(i) at a temperature of 12°C-22°C, or
(ii) at a temperature of 22°C-27°C followed by lowering the temperature of the cell culture to 12°C-22°C
(b) growing cells at a temperature of 12°C-22°C (c) optionally isolating the VLPs from the culture medium.

2. A method for the production of an immunogenic SAV VLP in a baculovirus based expression system, said method comprising:
(a) Infecting insect cells with said baculovirus expression vector either
(i) at a temperature of 12°C-22°C, or
(ii) at a temperature of 22°C-27°C followed by lowering the temperature of the cell culture to 12°C-22°C
(b) growing cells at a temperature of 12°C-22°C
(c) optionally isolating the VLPs from the culture medium wherein
the baculovirus expression vector comprises at least genetic information encoding an Alphavirus Capsid protein, E3 envelope protein, and an E2 envelope protein, 6K protein and E1 envelope protein, wherein at least the E2 and the E3 proteins are a Salmonid Alphavirus (SAV) E2 and E3 envelope proteins.

3. The use of claim 1, or the method of claim 2, **characterised in that** the step of growing the cells is performed at a temperature of 15°C-18°C.

4. The use of any one of claims 1, 3, ; or the method of any one of claims 2, 3, , wherein said expression vector comprises a polynucleotide encoding an Salmonid Alphavirus (SAV) Capsid protein, E3 protein, E2 envelope protein, 6K protein and E1 envelope protein.

5. Immunogenic Salmonid Alphavirus virus-like particle (SAV VLP directly obtained by the method according to any of claims 2-4.

6. Immunogenic SAV VLP according to claim 5 for use as a medicament.

7. Immunogenic SAV VLP according to claim 5 for use in the prevention of salmonid fish against SAV infection.

8. Vaccine for use in the prevention of salmonid fish against SAV infection, **characterised in that** said vaccine comprises an immunogenic amount of immunogenic SAV VLP's according to claim 5 and a pharmaceutically acceptable carrier.

9. Vaccine for use in the prevention of salmonid fish against SAV infection, **characterised in that** said vaccine comprises an immunogenic amount of insect cells comprising the SAV VLP according to claim 5 and a pharmaceutically acceptable carrier.

10. Vaccine for use in the prevention of salmonid fish against SAV infection according to claim 8 or 9, **characterized in that** said vaccine comprises an adjuvant.

11. Combination vaccine for use in the prevention of salmonid fish against SAV infection, **characterised in that** said vaccine comprises a vaccine according to any of claims 8-10 and at least one other vaccine against a fish-pathogenic microorganism or a fish-pathogenic virus.

12. Combination vaccine for use according to claim 11, **characterised in that** said fish-pathogenic microorganism or fish-pathogenic virus is selected from the group consisting of *Vibrio anguillarum, Aeromonas salmonicidae, Vibrio salmonicidae, Moritella viscosa, Vibrio ordalii, Flavobacterium sp., Flexibacter sp., Streptococcus sp., Lactococcus garvieae, Edwardsiella tarda, E. ictaluri, Piscirickettsia salmonis,* Viral Nervous Necrosis virus, Infectious Pancreatic Necrosis virus and iridovirus.

## Patentansprüche

1. Verwendung eines Baculovirus-Expressionsvektors bei einem Verfahren zur Herstellung eines immunogenen SAV-VLP in einem Expressionssystem auf Baculovirusbasis, **dadurch gekennzeichnet, dass** der Expressionsvektor wenigstens genetische Information umfasst, die ein Alphavirus-Capsidprotein, -E3-Hüllprotein und ein E2-Hüllprotein, 6K-Protein und E1-Hüllprotein codiert,
wobei es sich wenigstens bei den E2- und den E3-Proteinen um E2- und E3-Hüllproteine eines SAV (Salmonid Alphavirus) handelt und
wobei das Verfahren zur Herstellung des immunogenen SAV-VLP Folgendes umfasst:
(a) Infizieren von Insektenzellen mit dem Baculovirus-Expressionsvektor entweder
(i) bei einer Temperatur von 12°C-22°C oder
(ii) bei einer Temperatur von 22°C-27°C, gefolgt von Senken der Temperatur der Zellkultur auf 12°C-22°C,
(b) Kultivieren von Zellen bei einer Temperatur von 12°C-22°C, (c) gegebenenfalls Isolieren der VLP aus dem Kulturmedium.

2. Verfahren zur Herstellung eines immunogenen SAV-VLP in einem Expressionssystem auf Baculovirusbasis, wobei das Verfahren Folgendes umfasst:
(a) Infizieren von Insektenzellen mit dem Baculovirus-Expressionsvektor entweder
(i) bei einer Temperatur von 12°C-22°C oder
(ii) bei einer Temperatur von 22°C-27°C, gefolgt von Senken der Temperatur der Zellkultur auf 12°C-22°C,
(b) Kultivieren von Zellen bei einer Temperatur von 12°C-22°C,
(c) gegebenenfalls Isolieren der VLP aus dem Kulturmedium, wobei
der Baculovirus-Expressionsvektor wenigstens genetische Information umfasst, die ein Alphavirus-Capsidprotein, - E3-Hüllprotein und ein E2-Hüllprotein, 6K-Protein und E1-Hüllprotein codiert, wobei es sich wenigstens bei den E2- und den E3-Proteinen um E2- und E3-Hüllproteine eines SAV (Salmonid Alphavirus) handelt.

3. Verwendung nach Anspruch 1 oder Verfahren nach Anspruch 2, **dadurch gekennzeichnet, dass** der Schritt des Kultivierens der Zellen bei einer Temperatur von 15°C-18°C erfolgt.

4. Verwendung nach einem der Ansprüche 1, 3; oder Verfahren nach einem der Ansprüche 2, 3, wobei der Expressionsvektor ein Polynukleotid umfasst, das ein SAV(Salmonid Alphavirus)-Capsidprotein, -E3-Protein, - E2-Hüllprotein, -6K-Protein und -E1-Hüllprotein codiert.

5. Immunogenes SAV(Salmonid Alphavirus)-virusähnliches Partikel (SAV-VLP), erhältlich mit dem Verfahren gemäß einem der Ansprüche 2-4.

6. Immunogenes SAV-VLP gemäß Anspruch 5 zur Verwendung als Arzneimittel.

7. Immunogenes SAV-VLP gemäß Anspruch 5 zur Verwendung bei der Vorbeugung von Salmoniden gegen SAV-Infektion.

8. Impfstoff zur Verwendung bei der Vorbeugung von Salmoniden gegen SAV-Infektion, **dadurch gekennzeichnet, dass** der Impfstoff eine immunogene Menge immunogener SAV-VLP gemäß Anspruch 5 und einen pharmazeutisch unbedenklichen Träger umfasst.

9. Impfstoff zur Verwendung bei der Vorbeugung von Salmoniden gegen SAV-Infektion, **dadurch gekennzeichnet, dass** der Impfstoff eine immunogene Menge Insektenzellen, die das SAV-VLP gemäß Anspruch 5 umfassen, und einen pharmazeutisch unbedenklichen Träger umfasst.

10. Impfstoff zur Verwendung bei der Vorbeugung von Salmoniden gegen SAV-Infektion gemäß Anspruch 8 oder 9, **dadurch gekennzeichnet, dass** der Impfstoff ein Adjuvans umfasst.

11. Kombinationsimpfstoff zur Verwendung bei der Vorbeugung von Salmoniden gegen SAV-Infektion, **dadurch gekennzeichnet, dass** der Impfstoff einen Impfstoff gemäß einem der Ansprüche 8-10 und wenigstens einen anderen Impfstoff gegen einen fischpathogenen Mikroorganismus oder ein fischpathogenes Virus umfasst.

12. Kombinationsimpfstoff zur Verwendung gemäß Anspruch 11, **dadurch gekennzeichnet, dass** der fischpathogene Mikroorganismus bzw. das fischpathogene Virus ausgewählt ist aus der Gruppe bestehend aus *Vibrio anguillarum, Aeromonas salmonicidae, Vibrio salmonicidae, Moritella viscosa, Vibrio ordalii, Flavobacterium sp., Flexibacter sp., Streptococcus sp., Lactococcus garvieae, Edwardsiella tarda, E. ictaluri, Piscirickettsia salmonis,* VNN(Viral Nervous Necrosis)-Virus, IPN(Infectious Pancreatic Necrosis)-Virus und Iridovirus.

## Revendications

1. Utilisation d'un vecteur d'expression de baculovirus dans un procédé pour la production d'un SAV VLP immunogène dans un système d'expression à base de baculovirus,
**caractérisé en ce que** ledit vecteur d'expression comprend au moins des informations génétiques codant pour une protéine de capside, une protéine d'enveloppe E3 et une protéine d'enveloppe E2, une protéine 6K et une protéine d'enveloppe E1 d'alphavirus,
dans laquelle au moins les protéines E2 et E3 sont des protéines d'enveloppe E2 et E3 d'alphavirus de salmonidé (SAV) et
dans laquelle le procédé pour la production du SAV VLP immunogène comprend :
(a) l'infection de cellules d'insecte par ledit vecteur d'expression de baculovirus soit
(i) à une température de 12 °C à 22 °C, soit
(ii) à une température de 22 °C à 27 °C suivie par l'abaissement de la température de la culture de cellules jusqu'à 12 °C à 22 °C
(b) la culture de cellules à une température de 12 °C à 22 °C (c) facultativement l'isolement des VLP à partir du milieu de culture.

2. Procédé pour la production d'un SAV VLP immunogène dans un système d'expression à base de baculovirus, ledit procédé comprenant :
(a) l'infection de cellules d'insecte par ledit vecteur d'expression de baculovirus soit
(i) à une température de 12 °C à 22 °C, soit
(ii) à une température de 22 °C à 27 °C suivie par l'abaissement de la température de la culture de cellules jusqu'à 12 °C à 22 °C
(b) la culture de cellules à une température de 12 °C à 22 °C
(c) facultativement l'isolement des VLP à partir du milieu de culture, dans lequel
le vecteur d'expression de baculovirus comprend au moins des informations génétiques codant pour une protéine de capside, une protéine d'enveloppe E3, et une protéine d'enveloppe E2, une protéine 6K et une protéine d'enveloppe E1 d'alphavirus, dans lequel au moins les protéines E2 et E3 sont des protéines d'enveloppe E2 et E3 d'alphavirus de salmonidé (SAV).

3. Utilisation selon la revendication 1, ou procédé selon la revendication 2, caractérisé(e) en ce que l'étape de culture des cellules est effectuée à une température de 15 °C à 18 °C.

4. Utilisation selon l'une quelconque des revendications 1, 3, ou procédé selon l'une quelconque des revendications 2, 3, dans laquelle/lequel ledit vecteur d'expression comprend un polynucléotide codant pour une protéine de capside, une protéine E3, une protéine d'enveloppe E2, une protéine 6K et une protéine d'enveloppe E1 d'alphavirus de salmonidé (SAV).

5. Particule pseudovirale d'alphavirus de salmonidé (SAV VLP) immunogène, pouvant être obtenue par le procédé selon l'une quelconque des revendications 2 à 4.

6. SAV VLP immunogène selon la revendication 5 pour utilisation en tant que médicament.

7. SAV VLP immunogène selon la revendication 5 pour utilisation dans la prévention de poissons salmonidés contre une infection par SAV.

8. Vaccin pour utilisation dans la prévention de poissons salmonidés contre une infection par SAV, **caractérisé en ce que** ledit vaccin comprend une quantité immunogène de SAV VLP immunogènes selon la revendication 5 et un véhicule pharmaceutiquement acceptable.

9. Vaccin pour utilisation dans la prévention de poissons salmonidés contre une infection par SAV, **caractérisé en ce que** ledit vaccin comprend une quantité immunogène de cellules d'insecte comprenant le SAV VLP selon la revendication 5 et un véhicule pharmaceutiquement acceptable.

10. Vaccin pour utilisation dans la prévention de poissons salmonidés contre une infection par SAV selon la revendication 8 ou 9, **caractérisé en ce que** ledit vaccin comprend un adjuvant.

11. Vaccin combiné pour utilisation dans la prévention de poissons salmonidés contre une infection par SAV, **caractérisé en ce que** ledit vaccin comprend un vaccin selon l'une quelconque des revendications 8 à 10 et au moins un autre vaccin contre un microorganisme pathogène chez les poissons ou un virus pathogène chez les poissons.

12. Vaccin combiné pour utilisation selon la revendication 11, **caractérisé en ce que** ledit microorganisme pathogène chez les poissons ou virus pathogène chez les poissons est choisi dans le groupe constitué de *Vibrio anguillarum, Aeromonas salmonicidae, Vibrio salmonicidae, Moritella viscosa, Vibrio ordalii, Flavobacterium sp., Flexibacter sp., Streptococcus sp., Lactococcus garvieae, Edwardsiella tarda, E. ictaluri, Piscirickettsia salmonis,* virus de la nécrose nerveuse virale, virus de la nécrose pancréatique infectieuse et iridovirus.
